# EUROPEAN PATENT SPECIFICATION

(11) **EP 1 827 604 B1**
(45) Date of publication and mention of the grant of the patent: **20.11.2019**
(21) Application number: 05815888.2
(22) Date of filing: 12.12.2005
(51) Int. Cl.: A61P 35/00, A61K 31/12, A61K 39/44

(54) **METHODS AND COMPOSITIONS FOR ADOPTIVE IMMUNOTHERAPY**
VERFAHREN UND ZUSAMMENSETZUNGEN FÜR DIE ADOPTIVE IMMUNTHERAPIE
PROCEDES ET COMPOSITIONS POUR L' IMMUNOTHERAPIE ADOPTIVE

(30) Priority: 10.12.2004 AU 2004907072
(43) Date of publication of application: 05.09.2007
(60) Divisional application: 19151271.4
(73) Proprietor: Peter MacCallum Cancer Institute, East Melbourne VIC 3002 (AU)
(72) Inventor: TRAPANI, Joseph, A., Camberwell, VIC 3124 (AU); SMYTH, Mark, J., North Balwyn, VIC 3104 (AU); DARCY, Philip, K., East Melbourne, VIC 3002 (AU); KERSHAW, Michael, H., East Melbourne, VIC 3002 (AU)
(74) Representative: Mewburn Ellis LLP
(86) International application number: PCT/AU2005/001873
(87) International publication number: WO 2006/060878

(56) References cited:
- TENG MICHELE W L ET AL: "Immunotherapy of cancer using systemically delivered gene-modified human T lymphocytes." HUMAN GENE THERAPY JUL 2004, vol. 15, no. 7, July 2004 (2004-07), pages 699-708, XP002533909 ISSN: 1043-0342
- KERSHAW MICHAEL H ET AL: "Gene-engineered T cells as a superior adjuvant therapy for metastatic cancer." JOURNAL OF IMMUNOLOGY (BALTIMORE, MD. : 1950) 1 AUG 2004, vol. 173, no. 3, 1 August 2004 (2004-08-01), pages 2143-2150, XP002533910 ISSN: 0022-1767
- ALTENSCHMIDT U ET AL: "Adoptive transfer of in vitro-targeted, activated T lymphocytes results in total tumor regression." JOURNAL OF IMMUNOLOGY (BALTIMORE, MD. : 1950) 1 DEC 1997, vol. 159, no. 11, 1 December 1997 (1997-12-01), pages 5509-5515, XP002533911 ISSN: 0022-1767
- ALTENSCHMIDT U ET AL: "CYTOLYSIS OF TUMOR CELLS EXPRESSING IN THE NEU/ERBB-2, ERBB-3, AND ERBB-4 RECEPTORS BY GENETICALLY TARGETED NAIVE T LYMPHOCYTES" CLINICAL CANCER RESEARCH, THE AMERICAN ASSOCIATION FOR CANCER RESEARCH, US, vol. 2, no. 6, 1 June 1996 (1996-06-01), pages 1001-1008, XP001145747 ISSN: 1078-0432
- WILLEMSEN R ET AL: 'Redirecting human CD4+ T lymphocytes to the MHC class I-restricted melanoma antigen MAGE-A1 by TCR alphabeta gene transfer requires CD8alpha.' GENE THERAPY. vol. 12, no. 2, January 2005, pages 140 - 146, XP003022404
- MITSUYASU R T ET AL: 'Prolonged survival and tissue trafficking following adoptive transfer of CD4zeta gene-modified autologous CD4(+) and CD8(+) T cells in human immunodeficiency virus-infected subjects.' BLOOD. vol. 96, no. 3, August 2000, pages 785 - 793, XP002953226
- WALKER R E ET AL: 'Long-term in vivo survival of receptor-modified syngeneic T cells in patients with human immunodeficiency virus infection.' BLOOD. vol. 96, no. 2, 15 July 2000, pages 467 - 474, XP003022405
- MOELLER M ET AL: 'Adoptive transfer of gene-engineered CD4+ helper T cells induces potent primary and secondary tumor rejection.' BLOOD. vol. 106, no. 9, 01 November 2005, pages 2995 - 3003, XP003022406
- BAXEVANIS C N ET AL: "Targeting of tumor cells by lymphocytes engineered to express chimeric receptor genes", CANCER IMMUNOLOGY AND IMMUNOTHERAPY, SPRINGER-VERLAG, BERLIN, DE, vol. 53, no. 10, 1 January 2004 (2004-01-01), pages 893-903, XP009089635, ISSN: 0340-7004, DOI: 10.1007/S00262-004-0523-Y
- Hombach ET AL: "Tumor-specific T cell activation by recombinant immunoreceptors: CD3 zeta signaling and CD28 costimulation are simultaneously required for efficient IL-2 secretion and can be integrated into one combined CD28/CD3 zeta signaling receptor molecule.", The Journal of Immunology, vol. 167, no. 11, 1 December 2001 (2001-12-01), pages 6123-6131, XP055083123, ISSN: 0022-1767
- Pinthus J.H. and Eshhar Z.: "The T-body approach: towards cancer immuno-gene therapy" In: "Cancer immune Therapie: current and future strategies", 2002 ISBN: 3-527-60079-5 pages 287-298, * the whole document *
- MITSUYASU R T ET AL: "Prolonged survival and tissue trafficking following adoptive transfer of CD4zeta gene-modified autologous CD4+ and CD8+ T cells in human immunodeficiency virus-infected subjects", BLOOD, AMERICAN SOCIETY OF HEMATOLOGY, US, vol. 96, no. 3, 1 August 2000 (2000-08-01) , pages 785-793, XP002953226, ISSN: 0006-4971

## Description

### FIELD OF THE INVENTION

The present invention relates generally to the field of immunotherapy. More specifically, the invention relates to adoptive immunotherapy whereby immune cells of a patient are removed, engineered to trigger an immune response to a specific target antigen, and then administered to the patient. The invention provides novel methods and compositions that lead to improved immune responses to antigens such as those associated with tumors and infective agents.

### BACKGROUND TO THE INVENTION

The immune system is designed to target a large number of foreign antigens such as those presented by allergens and pathogens. Immune responses to specific antigens rely on the white bloods cells, and specifically the B cell and T cell populations. B cell responses typically result in the production of antibodies specific to the antigen while T cells are involved in cell mediated responses.

T cells are subclassified into two broad populations. Helper T cells are identified by the surface marker CD (Cluster Designation) 4, and are mainly involved in assisting in antibody production and induction of various immune responses. Cytotoxic T cells are distinguished by the CD8 marker and play an important role in recognizing, destroying and eliminating tumor cells and virus-infected cells, but do not produce an antibody specifically reacting with an antigen. Cytotoxic T cells directly recognize and act on antigens (typically antigenic peptides) from a target cell which are associated with major histocompatibility complex (MHC) Class I molecules present on the surface of the target cell membrane. The T cell receptor (TCR) existing on the surface of the cytotoxic T cell membrane specifically recognizes the above-mentioned antigenic peptides and MHC Class I molecules (and determines whether the antigenic peptide is "self" or "nonself"). Once a target cell is stably bound, and appropriate signalling takes place in the T cell, the target cell is then specifically destroyed and eliminated by the cytotoxic T cell.

Some current immunotherapeutic strategies involve the adoptive transfer to a patient of cytotoxic T cells engineered *in vitro* to have the ability to specifically react with an antigen of interest. While the adoptive immunotherapy has demonstrated promising results in the clinic particularly in patients with melanoma, there are considerable limitations to this method. A major problem is the lack of persistence of engineered cells at the disease site, in the blood or in a lymphoid organ, leading to incomplete eradication of the antigen-bearing cells. Also, very few of the re-infused cells are able to traffic accurately to tumor deposits. Another problem is that the immune response provided by adoptive immunotherapy methods of the prior art do not prevent the establishment of a new tumour or infection upon recurrence of the same tumor or infection.

To date, the application of adoptive immunotherapy has failed to demonstrate robust removal of cancerous cells, and is of even more limited use in refractory cancers such as breast, colon and ovarian carcinomas. Several factors have been proposed to account for the poor anti-tumor responses noted in the prior art, including down-regulation of MHC class I or class II/peptide from the cell surface (Garrido et al, 1993); and immunosuppressive effects of T immunoregulatory cells (Steitz et al, 2001; Shimizu et al, 1999).

It is an aspect of the present invention to alleviate or overcome a problem of the prior art by providing composition and methods leading to improved immune responses in adoptive immunotherapy.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application. The invention is defined by the claims and any other aspects or embodiments set forth herein not falling within the scope of the claims are for information only.

### SUMMARY OF THE INVENTION

Teng, M W L et al., Human Gene Therapy, Vol. 15(7), July 2004, pages 699-708, describes immunotherapy of cancer using systemically delivered gene-modified human T lymphocytes.

Kershaw, M H et al., J. Immunology Vol. 173(3), 1 August 2004, pages 2143-2150, describes gene-engineered T cells as a superior adjuvant therapy for metastatic cancer.

Altenschmidt U et al., J. Immunology Vol. 159(11), 1 December 1997, pages 5509-5515, reports that adoptive transfer of *in vitro*-targeted, activated T lymphocytes results in total tumor regression.

Altenschmidt U et al., Clinical Cancer Research, Vol. 2(6), 1 June 1996, pages 1001-1008, describes cytolysis of tumor cells expressing the NEW/ERBB-2, ERBB-3 and ERBB-4 receptors by genetically targeted naïve T lymphocytes.

In a first aspect the present invention provides a composition for producing an adoptive immune response to a target cell in a subject, said composition including a CD4+ T cell engineered to express a molecule capable of binding to an antigen on the target cell, and a CD8+ T cell engineered to express a molecule capable of binding to an antigen on the target cell; wherein the ratio of CD4+ T cells to CD8+ T cells is greater than about 1:3 and less than about 3:1.

Applicants have found that in adoptive transfer experiments *in vivo,* administration of sufficient numbers of antigen engineered CD4+ in combination with engineered CD8+ cells leads to a significantly enhanced immune response to a target cell. It is demonstrated herein that the injection of CD4+ and CD8+ T cells engineered to express a molecule capable of binding to a tumor associated antigen led to significant improvement in survival of subjects having established lung metastases, compared with transfer of unfractionated spleen-derived (largely CD8+) engineered T cells. Importantly, anti-tumor responses in the subjects correlated with persistence and localization of engineered T cells at the tumor site and in the blood and spleen. Applicants have further demonstrated herein that subjects treated with engineered CD4+ and CD8+ T cells that survived primary tumor challenge could reject a subsequent rechallenge with the same tumor. The studies disclosed herein demonstrate the therapeutic advantage in the combined transfer of antigen-specific gene-modified CD4+ and CD8+ T cells to significantly enhance T cell adoptive strategies for the treatment conditions such as cancer and infectious diseases.

In a preferred embodiment of the method, the adoptive immune response involves the interaction of the engineered T cells with the target cell wholly in an *in vivo* environment. Applicants demonstrate herein for the first time the adoptive transfer of engineered CD4+ T cells and T CD8+ cells directly into a subject, the T cells trafficking to the target cell, with all three cells interacting leading to an adoptive immune response. By contrast, methods described in the prior art require that the engineered T cells and target cell interact in an *in vitro* environment before administration into the subject. The methods described by the prior art are not clinically applicable, and serve only to demonstrate the basic principle of adoptive cell transfer.

Furthermore, in generating an improved adoptive immune response Applicants have shown that increasing the proportion of engineered CD4⁺ T cells could more effectively eradicate disease.

Preferably, the effective number of engineered CD4+ cells is greater than a number selected from the group consisting of about 1 x 10⁶, about 2.5 x 10⁶, about 5 x 10⁶, about 7.5 x 10⁶, and about 9 x 10⁶. Where the subject is an adult human, between about 10⁸ and about 10¹² CD4+ cells may be used.

Preferably, the effective number of engineered CD8+ cells is greater than a number selected from the group consisting of about 1 x 10⁶, about 2.5 x 10⁶, about 5 x 10⁶, about 7.5 x 10⁶, and about 9 x 10⁶. Where the subject is an adult human, between about 10⁸ and about 10¹² CD8+ cells may be used.

In a highly preferred embodiment of the method the effective number of engineered CD4+ cells is about 5 x 10⁶ and the effective number of engineered CD8+ cells is about 5 x 10⁶. Where the subject is an adult human, between about 10¹⁰ and 10¹¹ each of CD4+ and CD8+ cells may be used.

While the Applicants have identified effective numbers of CD4+ cells, it was also considered that the ratio of engineered CD4+ to CD8+ cells may be important. In a preferred method the ratio of CD4+ cells to CD8+ cells administered to the subject is greater than about 1:9 and less than about 9:1. More preferably the ratio is greater than about 1:3 and less than about 3:1. In a highly preferred embodiment of the method the ratio is about 1:1.

Preferably the molecule capable of binding to the antigen includes sequences from TCR-ζ and/or CD28 co-stimulatory signalling domains.

-The skilled person will understand- that the present invention is essentially generic and will be useful for directing a cell mediated immune response against a broad range of antigens such as those found in or on tumor cells, or antigens found in or on cells infected with a virus (for example HIV), a Mycobacterium (e.g. *Mycobacterium tuberculosis*) or a parasite (e.g. *Plasmodium falciparum*)*.* However, in a preferred embodiment of the method the antigen is an antigen associated with a cancerous cell.

Preferably the engineered cells persist locally at the tumor or infection site in the subject long term. In one form of the method the engineered cells persist at the tumour or infection site for at least 100 days.

In a preferred form of the method, the adoptive immune response leads to the complete eradication of the infection or tumor.

While a number of forms of administration of the cells will be acceptable, a preferred form of the method provides that administration is by systemic injection. The ability to administer the cells systemically reveals a further advantage of the present method in that the Applicants have demonstrated the ability of the cells to travel to and deposit at the site of infection or tumour or other pathology from a remote location. In contrast to the prior art the Applicants have demonstrated complete eradication of established tumor at a distant site following systemic injection of transduced mouse CD8⁺ and CD4⁺ T cells. In addition, the studies demonstrate long-term persistence and function of these gene-engineered T cells after tumor rechallenge.

Preferably the adoptive immune response is capable of preventing establishment of a new tumor or infection. The experiments described herein demonstrate for the first time *in vivo* that the provision of engineered antigen-specific CD4⁺ T cells can significantly improve anti-tumor responses, maintain persistence of transferred T cells and induce a potent recall response after tumor rechallenge. This is a substantial advance over the adoptive immunotherapy methods of the prior art.

In another aspect the present invention provides a composition for adoptive immunotherapy including an engineered CD4+ T cell and an engineered CD8+ T cell.

In another aspect the present invention provides a composition for adoptive immunotherapy consisting of an engineered CD4+ T cell and an engineered CD8+ T cell. Applicants have surprisingly found that it is not necessary to include a target cell in the composition before administration to the subject. This is of considerable advantage since it is not necessary to remove tissue containing a target cell from the subject.

In yet a further aspect the present disclosure provides a method for treating or preventing a cancer or an infection, said method including administering to a animal in need thereof an engineered CD4+ T cell and an engineered CD8+ T cell wherein the CD4+ T cell and CD8+ T cell are engineered to express a molecule capable of binding to an antigen of the cancer or infection. It should be understood that the method is essentially generic in nature and may be applied to many forms of cancer or infection.

In yet a further aspect the present disclosure provides a method for treating or preventing a cancer or an infection, said method including administering to a animal in need thereof a composition described herein.

In yet a further aspect the present disclosure provides a method for treating or preventing a cancer or an infection, said method including a method for producing an adoptive immune response described herein.

### BRIEF DESCRIPTION OF THE FIGURES

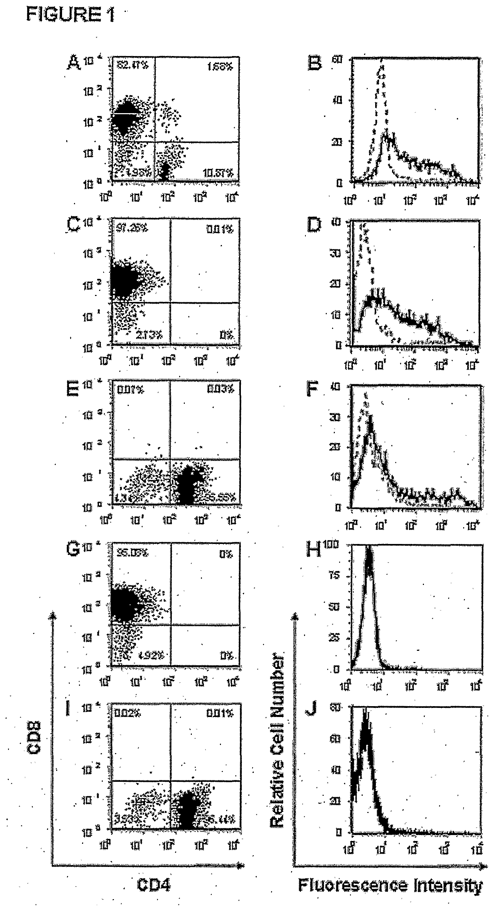

**FIG 1** shows expression of the scFv-CD28-ζ receptor in transduced CD8⁺ and CD4⁺ primary mouse T cells. Splenic T cells enriched from BALB/c mice were depleted into CD4⁺ and CD8⁺ T cells subsets or left as unfractionated T cells prior to retroviral transduction with the scFv-CD28-ζ receptor. Transduced unfractionated T cells consisted of 80-85% CD8⁺ T cells and ∼10% CD4⁺ T cells (A) while isolated populations consisted of greater than 90% CD8⁺ (C and G) or CD4⁺ T-cells (E and I). Chimeric scFv receptor expression was detected in unfractionated transduced T cells (B), CD8⁺ (D) and CD4⁺ (F) T cells by flow cytometry following staining with an anti-tag mAb and PE-labeled sheep anti-mouse Ig (solid line) or with the PE-labeled secondary alone (dashed line). Negligible receptor expression was detected in isolated populations of CD8⁺ (H) and CD4⁺ (J) T cells transduced with the empty LXSN vector. Similar results were obtained in ten experiments.

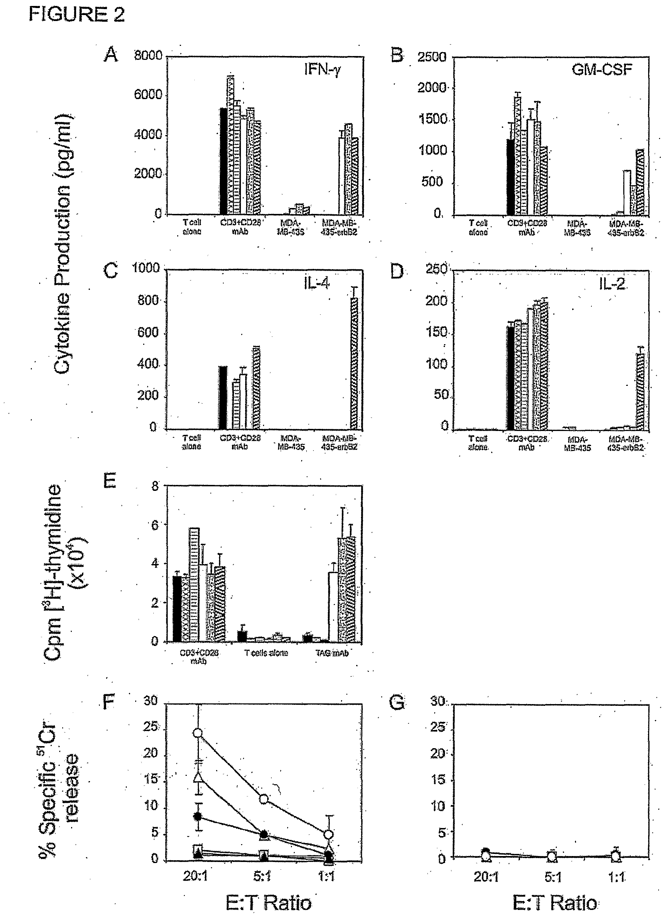

**FIG 2** shows antigen-specific cytokine secretion, proliferation and cytotoxicity by transduced mouse CD8⁺ and CD4⁺ T cells. To evaluate cytokine secretion, unfractionated T cells (open bars), CD8⁺ T cells (dotted bars) and CD4⁺ T cells (diagonally striped bars) transduced with the scFv-CD28-ζ receptor or mock transduced unfractionated T cells (closed bars), CD8⁺ T cells (cross-hatched bars) and CD4⁺ T cells (horizontally striped bars) were co-cultured with media alone or irradiated MDA-MB-435-erbB2 cells or MDA-MB-435 parental cells or stimulated with plate-bound anti-CD3 and -CD28 mAbs in 12-well plates for 24 hrs. Supernatants were harvested and assessed for IFN-γ (A), GM-CSF (B), IL-4 (C) and IL-2 (D) production by ELISA. Results are expressed as pg/ml of cytokine secreted ± SE for duplicate samples of three representative experiments. The proliferative capacity of each transduced T cell population was evaluated in a 72 hr [³H]-thymidine incorporation assay (E). Transduced unfractionated T cells (open bars), CD8⁺ T cells (dotted bars) and CD4⁺ T cells (diagonally striped bars) or mock transduced unfractionated T cells (closed bars), CD8⁺ T cells (cross-hatched bars) and CD4⁺ T cells (horizontally striped bars) were incubated with media alone or stimulated with plate-bound anti-CD3 and -CD28 mAbs or anti-tag mAb for 3 days. Results are expressed as means ± SE of triplicate samples and are representative of three experiments. Specific cytolytic function of scFv-CD28-ζ transduced mouse T cells was evaluated in a 6 hr ⁵¹Cr release assay. Transduced unfractionated (open triangles) and CD8⁺ (open circles) T cells demonstrated greater lysis of MDA-MB-435-erbB2 target cells than CD4⁺ (closed circles) transduced T cells (F). No lysis of MDA-MB-435 parental target cells was observed by chimeric receptor transduced T cells (G) and mock transduced unfractionated T cells (closed squares), CD8⁺ T cells (closed triangles) or CD4⁺ T cells (open squares) did not lyse either cell line. The spontaneous lysis was < 10% in all assays. Results are expressed as % specific ⁵¹Cr release ± SE (%) of triplicate samples representative of at least two experiments.

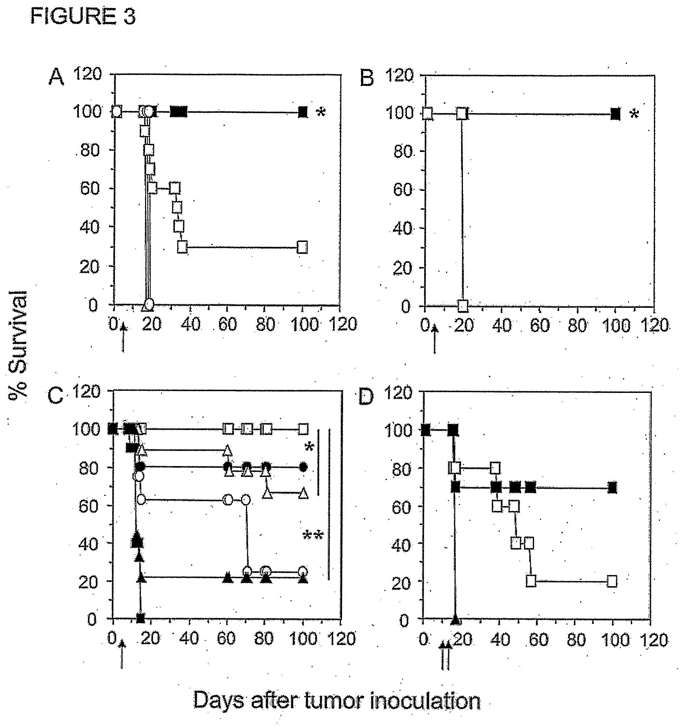

FIG 3 shows increased transfer of engineered CD4⁺ T cells enhances tumor-free survival of mice. (A) Groups of 10 scid mice were injected i.v. with 5x10⁶ MDA-MB-435-erbB2 cells at day 0 prior to i.v. injection of scFv-CD28-ζ transduced T cells at day 5. Mice treated with a 1:1 ratio of CD4⁺ (5x10⁶) and CD8⁺ (5x10⁶) transduced T cells (closed squares) demonstrated 100% survival compared to 30% survival of mice receiving unfractionated transduced T cells (open squares) (**p*<0.005, Mann-Whitney test). No survival of mice was observed receiving CD4⁺ transduced T cells alone (closed circles), CD8⁺ transduced T cells alone (open circles), control transduced T cells (open triangles) or no T cells (closed triangles) (B) Enhanced survival of mice was due to increased transfer of antigen-specific CD4⁺ T cells. All scid mice bearing 5 day MDA-MB-435-erbB2 tumor that received a 1:1 ratio of CD8⁺ (5 x 10⁶) and CD4⁺ (5 x 10⁶) transduced T cells (closed squares) survived compared with mice receiving either a 1:1 combination of transduced CD8⁺ T cells (5 x 10⁶) and naïve CD4⁺ T cells (5 x 10⁶) (open squares), 1:1 combination of transduced CD8⁺ (5 x 106) T cells and CD4⁺ T cells (5 x 10⁶) transduced with an irrelevant scFv-anti-CEA-γ receptor (open triangles) or no T cell treatment (closed triangles) (**p*<0.0001, Mann-Whitney test) (C) To determine the optimal ratio of transduced CD4⁺ and CD8⁺ T cells required to achieve 100% survival of mice bearing 5 day established MDA-MB-435-erbB2 lung metastases, mice were treated with transduced CD8⁺ and CD4⁺ T cells at the following ratios; 1:1 (5 x 10⁶ CD8⁺, 5 x 10⁶ CD4⁺ T cells) (open squares), 9:1 (9 x 10⁶ CD8⁺, 1 x 10⁶ CD4⁺ T cells) (closed triangles), 3:1 (7.5 x 10⁶ CD8⁺, 2.5 x 10⁶ CD4⁺ T cells) (open triangles), 1:3 (2.5 x 10⁶ CD8⁺, 7.5 x 10⁶ CD4⁺ T cells) (closed circles) or 1:9 (1 x 10⁶ CD8⁺, 9 x 10⁶ CD4⁺ T cells (open circles). Control mice were treated with mock transduced CD4⁺ and CD8⁺ T cells at a 1:1 ratio (closed squares) (**p*<0.05, ***p*<0.0005, Mann-Whitney test) (D) Increased transfer of engineered CD4⁺ T cells enhanced survival of mice bearing 10 day MDA-MB-435-erbB2 tumor. Scid mice received two i.v. injections of transduced CD8⁺ and CD4⁺ T cells at day 10 (5 x 10⁶ CD8⁺, 5 x 10⁶ CD4⁺ T cells) and day 12 (2.5 x 10⁶ CD8⁺, 2.5 x 10⁶ CD4⁺ T cells) (closed squares) or unfractionated transduced T cells at day 10 (10⁷) and day 12 (5 x 10⁶) (open squares). Control mice received 1:1 transfer of scFv-anti-CEA-y transduced CD8⁺ and CD4⁺ T cells (closed triangles). All results are calculated as the percentage of each group surviving and are representative of two experiments performed. Arrows depict days of T-cell transfer.

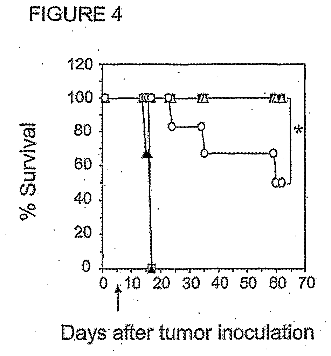

**FIG 4** shows antigen-specific release of IFN-γ by transduced CD8⁺ T cells is critical for enhanced survival of mice. Scid mice were injected i.v. with 5 × 10⁶ MDA-MB-435-erbB2 tumor cells followed by combined transfer of transduced CD8⁺ (5 x 10⁶) and CD4⁺ (5 x 10⁶) donor T cells at day 5 from BALB/c wildtype or BALB/c IFN-γ^{-/-} mice. Mice received either 1:1 transfer of scFv-CD28-ζ transducedCD8⁺IFN-γ^{+/+}:CD4⁺IFN-γ^{+/+} T cells (closed squares), CD8⁺IFN-γ^{-/-} :CD4⁺IFN-γ^{-/-} T cells (closed triangles), CD8⁺IFN-γ^{-/-}:CD4⁺IFN-γ^{+/+} T cells (open circles) or CD8⁺IFN-γ^{+/+}:CD4⁺IFN-γ^{-/-} (open triangles) T cells. Control mice received a 1:1 transfer of wildtype CD8⁺ and CD4⁺ T cells transduced with the scFv-anti-CEA-y receptor (open squares). Results are calculated as the percentage of each group surviving and are representative of 2 experiments. Arrows depict the day of T-cell transfer (**p*<0.05, Mann-Whitney test).

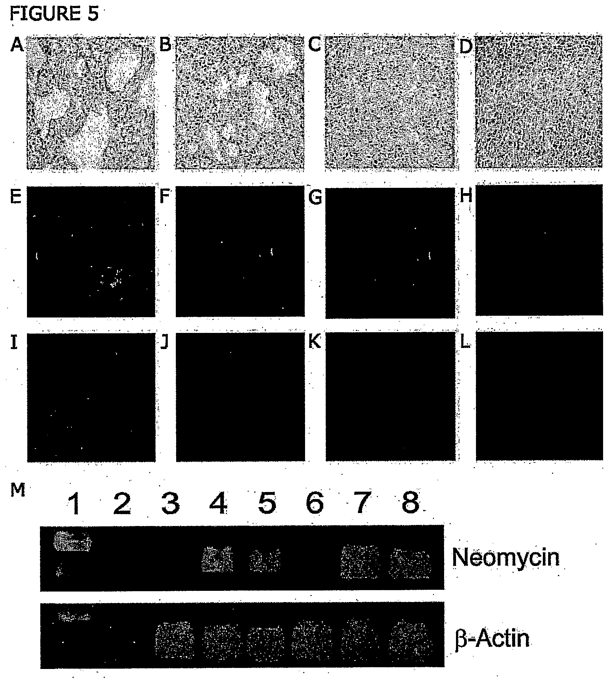

**FIG 5** shows detection of transduced CD8⁺ and CD4⁺ T cells in vivo. Scid mice were injected i.v. with MDA-MB-435-erbB2 tumor cells (5 x 10⁶ cells) at day 0 followed by i.v. injection of transduced T cells at day 5. Lungs were harvested and prepared for immunohistological analysis 16 days after tumor inoculation. Lung sections from mice that received 1:1 CD4⁺ (5 x 10⁶) and CD8⁺ (5 x 10⁶) scFv-CD28-ζ transduced T cells (A, E and I), unfractionated transduced T cells (10⁷), mouse #1 (B, C and F), mouse #2 (G, J and K), or 1:1 CD4⁺ (5 x 10⁶) and CD8⁺ (5 x 10⁶) scFv-α-CEA-γ transduced T cells (D, H and L) were stained by H&E, with anti-CD4 (green), anti-CD8 (red) and anti-CD11b (blue) mAbs (E-H) or with anti-tag (red) and anti-CD11b (green) mAbs (I-L). Representative fields of multiple sections are shown. Magnification, x400. (M) Peripheral blood and spleens were harvested from mice at day 100 and used for detection of neomycin phosphotransferase mRNA (∼400 bp). Both peripheral blood (lanes 4 and 5) and spleens (lanes 7 and 8) of two representative mice treated with scFv-CD28-ζ-transduced CD4⁺ and CD8⁺ T cells-demonstrated presence of the neomycin phosphotransferase gene. There was no neomycin phosphotransferase detected in either the peripheral blood (lane 3) or spleens (lane 6) of normal scid control mice. As controls, β-actin was detected in all peripheral blood (lane 3) and spleen samples (lane 8) tested. No neomycin phosphotransferase or β-actin were detected in empty DNA controls (lane 2). Lane 1 = 1 kb Plus markers.

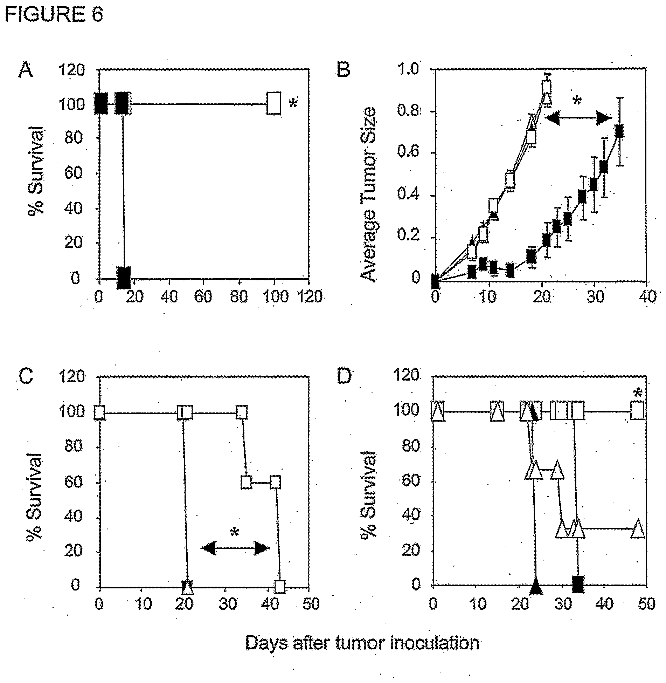

**FIG 6** shows long-term surviving mice can induce an antigen-specific secondary response to tumor rechallenge. (A) Long-term surviving mice (100 days post primary MDA-MB-435-erbB2 tumor inoculation) treated with combined transfer of CD4⁺ and CD8⁺ transduced T cells were able to reject a secondary i.v. injection of MDA-MB-435-erbB2 tumor cells (5 x 10⁶ cells) (open squares). Normal mice did not reject i.v. injection of MDA-MB-435-erbB2 tumor cells (5 x 10⁶) (closed squares) (**p* < 0.0001, Mann-Whitney test). (B) As a further test of specificity, long-term surviving mice were subcutaneously injected with 5x10⁴ mouse mammary carcinoma 4T1.2-erbB2 (closed squares) or 4T1.2-parental cells (open squares) and their growth was statistically compared (**p*<0.005, Mann-Whitney test). As controls, normal scid mice received either 5 x 10⁴ 4T1.2-erbB2 (closed triangles) or 4T1.2-parental tumor cells (open triangles) (C) Survival of long-term surviving mice subcutaneously injected with 5x10⁴ 4T1.2-erbB2 (open squares) or 4T1-parental (open triangles) was compared (**p*<0.005, Mann-Whitney test). As controls, normal scid mice were challenged with 5 x 10⁴ 4T1.2-erbB2 (closed squares) or 4T1-parental tumor cells (closed triangles) (D) To test the ability of mice to eradicate a lower dose of 4T1.2-erbB2 tumor, long-term surviving mice were challenged subcutaneously with either 5 x 10⁴ cells (open triangles) or 5 x 10³ 4T1.2-erbB2 tumor cells (open squares) and survival was statistically compared (**p*<0.001, Mann-Whitney test). As controls the growth of 4T1.2-erbB2 tumor cells in normal SCID mice at 5 x 10⁴ cells (closed triangles) or 5 x 10³ cells (closed squares) was performed. All results are calculated as the percentage of each group surviving or represented as the mean size (mm²) ± SEM and are representative of 2 experiments.

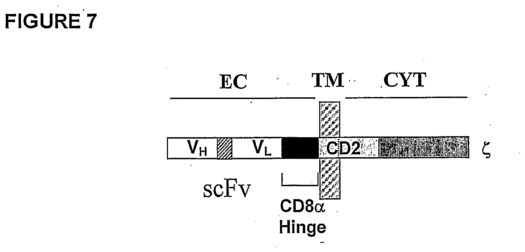

**FIG 7** shows a schematic representation of the scFv-CD28- receptor. The construct consists of the extracellular (EC) V_{H} and V_{L} regions of the scFv antibody joined by a flexible linker, a membrane-proximal CD8α hinge region and the transmembrane (TM) and cytoplasmic (CYT) regions of the CD28 signaling chain fused to the intracellular domain of T cell receptor ζ chain. A c-myc tag epitope is incorporated into the C-terminus of the V_{L} region for expression analysis.

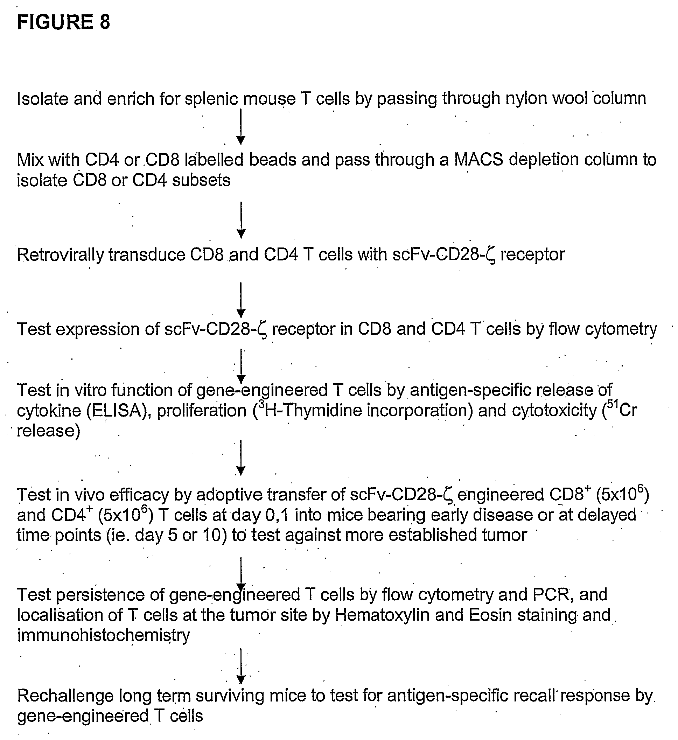

**FIG 8** shows a flow diagram describing generation of gene-engineered CD8⁺ and CD4⁺ T cells and their functional characterisation in vitro and in vivo.

### DETAILED DESCRIPTION OF THE INVENTION

In a first aspect the present disclosure provides a method for producing an adoptive immune response to a target cell in a subject, said method including the steps of engineering a CD4+ T cell and engineering a CD8+ T cell to express a molecule capable of binding to an antigen on the target cell, and administering an effective number of the engineered CD4+ and CD8+ T cells to the subject.

Applicants have surprisingly found that in adoptive transfer experiments *in vivo,* administration of sufficient numbers of antigen engineered CD4+ in combination with engineered CD8+ cells leads to a significantly enhanced immune response to a target cell. It is demonstrated herein that the injection of CD4+ and CD8+ T cells engineered to express a molecule capable of binding to a tumor associated antigen led to significant improvement in survival of subjects having established lung metastases, compared with transfer of unfractionated spleen-derived (largely CD8+) engineered T cells. Importantly, anti-tumor responses in the subjects correlated with persistence and localization of engineered T cells at the tumor site, and in the blood and spleen. Applicants have further demonstrated herein that subjects treated with engineered CD4+ and CD8⁺ T cells that survived primary tumor challenge could reject a subsequent rechallenge with the same tumor. The studies disclosed herein demonstrate the therapeutic advantage in the combined transfer of antigen-specific gene-modified CD4+ and CD8+ T cells to significantly enhance T cell adoptive strategies for the treatment conditions such as cancer and infectious diseases.

As used herein, the term "adoptive immune response" is intended to include any immune response elicited in an animal as a result of introducing or reintroducing to the animal a cell of the immune system that has been modified ex *vivo* (that is, outside the animal) in any way to enhance participation in an immune activity in the animal.

In a preferred embodiment of the method, the adoptive immune response involves the interaction of the engineered T cells with the target cell wholly in an *in vivo* environment. Applicants demonstrate herein for the first time the adoptive transfer of engineered CD4+ T cells and T CD8+ cells directly into a subject, the T cells trafficking to the target cell, with all three cells interacting leading to an adoptive immune response. By contrast, methods described in the prior art require that the engineered T cells and target cell interact in an *in vitro* environment before administration into the subject. The methods described by the prior art are not clinically applicable, and serve only to demonstrate the basic principle of adoptive cell transfer.

Furthermore, in generating an improved adoptive immune response Applicants have shown that increasing the proportion of engineered CD4⁺ T cells could more effectively eradicate disease. A retroviral transduction system for the expression of the scFv-CD28-ζ receptor was used to create an engineered T cell reactive against the erbB2 tumour antigen, on the surface of both CD8⁺ and CD4⁺ splenic mouse T cells. Strikingly, increasing the number of CD4+ cells above that normally produced when a splenic preparation of unfractionated T cells is used led to complete eradication of established tumors in mice, whereas transfer of mainly CD8⁺ T cells (i.e. with low numbers of CD4+ cells) was significantly less effective.

Applicants propose a number of possible mechanisms to account for the results obtained, and it may be that a combination of these mechanisms or indeed other mechanisms are involved. It should be understood that these proposed theories do not limit the generality of the invention as described herein. Overall, it is-postulated that the engineered CD4+ and CD8+ cells travel to the tumour or infection site and interact with each other, and the target cell, to provide enhanced conditions for cytotoxic activity in the local environment of the target cell.

It appears that the enhanced efficacy was dependent at least in part on the antigenic-specificity of the CD4⁺ T cells, and correlated with tumor localization and long-term persistence of both CD8⁺ and CD4⁺ T cells. Importantly, mice "cured" of primary tumor could subsequently reject a secondary challenge with erbB2⁺ tumor. This is the first study to (i) definitively demonstrate in vivo a role for engineered antigen-specific CD4⁺ T cells for enhancing the anti-tumor response (ii) demonstrate long-term persistence of adoptively transferred engineered T cells and (iii) demonstrate a potent antigen-specific secondary response by gene-engineered T cells following tumor rechallenge.

More specifically the provision of an effective number of CD4+ and CD8+ antigen-specific T cells may facilitate the T "helper" function. Interestingly, scFv-transduced CD8⁺ T cells could specifically mediate high levels of cytotoxicity and IFN-γ release in vitro but no IL-2 secretion following antigen ligation. In contrast, scFv-transduced CD4⁺ T cells demonstrated reduced cytotoxicity but high levels of both IFN-γ and IL-2 secretion. Applicants were able to demonstrate in vivo using donor T cells from IFN-γ^{-/-} mice that IFN-γ released by engineered CD4⁺ T cells could contribute to the anti-tumor effect in the absence of IFN-γ from CD8⁺ T cells. Nevertheless, given the fact that IL-2 has been previously shown to be important for maintaining CD8⁺ T cell function in vivo (Theze et al, 2004; Taniguchi and Minami 1993), it remains possible that the provision of IL-2 'help' from transduced CD4⁺ T cells may also play a role in the improved anti-tumor efficacy observed following combined transfer of both engineered CD8⁺ and CD4⁺ T cells.

Accordingly, in a preferred form of the method, the adoptive immune response involves CD4+ T cell help. The issue of whether the addition of antigen-specific engineered CD4⁺ T cell help (ie. IL-2 release) may enhance anti-tumor immunity, long-term persistence and secondary tumor rejection has never been considered a relevant question for adoptive transfer of T cells *in vivo*. A major problem is that cytokines such as IL-2 are toxic when administered systemically at clinically effective doses. Applicants' approach obviates the need to administer exogenous cytokines to enhance the immune response against a target cell, given the very precise manner in which IL-2 is apparently directed to the tumour or infection site. Given that the art provides a number of methods for efficiently transducing both CD8⁺ and CD4⁺ human T cells (Hombach et al, 2001b; Teng et al, 2004; Finney et al, 2004; Hombach et al, 2001a) adoptive immunotherapy using T cells is now clinically feasible. The studies described herein demonstrate the importance of engineered CD4⁺ T cell help represents a significant step for enhancing redirected cytotoxic T cell therapy for the treatment of cancer and infectious diseases.

Another theory is that the adoptive immune response provided by using engineered CD4+ and CD8+ T cells may rely at least to some extent on the release of IFN-γ by the CD8+ cells. Accordingly, in a preferred form of the method, the adoptive immune response involves the release of IFN-γ by the engineered CD8+ T cells. To demonstrate the importance of antigen-specific release of IFN-γ secreted by either the CD8⁺ cells, Applicants transduced and adoptively transferred different combinations of CD8⁺ and CD4⁺ donor T cells from BALB/c wildtype and IFN-γ knockout mice. All 5 mice bearing tumor survived following treatment with 1:1 transfer of IFN-γ wildtype CD8⁺ and CD4⁺ transduced T cells (Fig. 4). This was also found to be the case with mice treated with 1:1 transfer of CD8⁺IFN-γ^{+/+} and CD4⁺IFN-γ^{-/-} transduced T cells suggesting that IFN-γ produced by engineered CD4⁺ T cells was not critical for this anti-tumor effect. In contrast, only 50% of mice treated with CD8⁺IFN-γ^{-/-} and CD4⁺IFN-γ^{+/+} transduced T cells survived indicating that IFN-γ secreted by engineered CD8⁺ T cells was absolutely essential to achieve 100% survival of mice (Fig. 4). Nevertheless, IFN-γ produced by engineered antigen-specific CD4⁺ T cells was able to partially compensate given the fact that transfer of engineered CD8⁺IFN-γ^{-/-} and CD4⁺IFN-γ^{-/-} T cells had no effect. As a further control, mice treated with IFN-γ^{+/+} CD4⁺ and CD8⁺ T cells transduced with an irrelevant receptor were unable to eradicate tumor (Fig. 4).

Preferably, the effective number of engineered CD4+ cells is greater than a number selected from the group consisting of about 1 x 10⁶, about 2.5 x 10⁶, about 5 x 10⁶, about 7.5 x 10⁶, and about 9 x 10⁶. Where the subject is an adult human, between about 10⁸ and about 10¹² CD4+ cells may be used.

Preferably, the effective number of engineered CD8+ cells is greater than a number selected from the group consisting of about 1 x 10⁶, about 2.5 x 10⁶, about 5 x 10⁶, about 7.5 x 10⁶, and about 9 x 10⁶. Where the subject is an adult human, between about 10⁸ and about 10¹² CD8+ cells may be used.

In a highly preferred embodiment of the method the effective number of engineered CD4+ cells is about 5 x 10⁶ and the effective number of engineered CD8+ cells is about 5 x 10⁶. Where the subject is an adult human, between about 10¹⁰ and 10¹¹ each of CD4+ and CD8+ cells may be used.

It will be appreciated that the number of CD4+ and CD8+ cells administered may need to be optimized depending on the subject being treated. Once the skilled person has been availed of the finding provided by the Applicants that an effective number of engineered CD4+ cells must be used for the efficient trafficking of adoptive immunotherapy *in vivo,* a process of routine experimentation could be used to identify an effective number. For example, an animal such as a mouse may require less cells than a larger animal such as a human. Without wishing to place any undue limitation on the present invention, the skilled person could extrapolate the effective number found for a mouse to estimate the effective number for an adult human. For example, if a 20g mouse required the administration of 5 x 10⁶ cells, then an adult human weighing 80 kg may require 5 x 10⁶ x (80000/20) = 2 x 10¹⁰ cells. In a preferred form of the method, where the subject is an adult human, between about 10⁸ and about 10¹² each of CD4+ and CD8+ cells are used

While the Applicants have identified effective numbers of CD4+ cells, it was also considered that the ratio of engineered CD4+ to CD8+ cells may be important. To determine the optimal ratio of scFv-CD28-ζ transduced CD4⁺ and CD8⁺ T cells required to achieve 100% survival, mice bearing 5 day MDA-MB-435-erbB2 tumor were treated with different ratios of scFv-CD28-ζ transduced CD8⁺ and CD4⁺ T cells. Interestingly, only a 1:1 ratio of transduced CD8⁺ and CD4⁺ T cells (5 x 10⁶ of each T cell subset) resulted in 100% survival consistent with previous experiments (Fig. 3 C). In contrast, the percentage of survivors decreased significantly when mice received a greater proportion of either CD8⁺ or CD4⁺ transduced T cells (Fig. 3 C). Accordingly, in a preferred method the ratio of CD4+ cells to CD8+ cells administered to the subject is greater than about 1:9 and less than about 9:1. More preferably the ratio is greater than about 1:3 and less than about 3:1. In a highly preferred embodiment of the method the ratio is about 1:1. As will be appreciated, once the skilled person is availed of the information that the ratio of engineered CD4+ T cells to engineered CD8+ T cells may be important to the efficacy of the method, it would be routine to investigate the optimal ratio for any species, subspecies, or individual.

The ability of adoptively transferred engineered CD8⁺ and CD4⁺ T cells to control more advanced tumors was also tested. Mice bearing 10 day MDA-MB-453-erbB2 tumor that received two i.v. injections of CD4⁺ and CD8⁺ transduced T cells at a 1:1 ratio (7.5 x 10⁶ total cells of each subset) demonstrated a 70% survival rate (Fig. 3 D). In contrast, only 20% of mice treated with unfractionated transduced T cells survived (Fig. 3 D). Collectively, these results demonstrated for the first time in vivo that optimal transfer of engineered antigen-specific CD4⁺ T cells could significantly improve redirected T cell therapy for treatment of cancer.

The skilled person will be familiar with a number of sources for T cells useful in the context of the present invention. Tissues involved in specific immune responses such as the spleen, thymus, lymph node, bone marrow, or the blood may be used. Alternatively, tumour or infected tissue could be used as these tissues will contain CD4+ and CD8+ cells useful in the context of the present invention. For human therapy it is contemplated that-blood or tumour is the more appropriate source.

Where the source of T cells is the spleen, the ratio of CD4+ cells to CD8+ cells will typically need to be adjusted to provide an effective number of CD4+ T cells. Generally, an unfractionated T cell population derived from the spleen will consist of 80 to 85% CD8+ cells and about 10 to 15% CD4+ cells. Accordingly, it may be necessary to increase the level of CD4+ cells compared with CD8+ cells. The skilled person will be familiar with a number of methods suitable for altering the number of CD4+ cells in a cell population. Methodologies such fluorescence-activated cell sorting (FACS), magnetic beads coated with CD4+ specific antibody, antibody "panning", complement and monoclonal antibody depletion are contemplated to be useful.

Preferably the molecule capable of binding to the antigen includes a receptor for the antigen, or a portion of a receptor or a functional equivalent of a receptor. The receptor could be a protein or a glycoprotein having the required primary and/or secondary and/or tertiary structure providing a suitable steric and/or electrostatically charged surface by which the antigen may bind. The receptor may be based on a receptor that is known to bind the antigen in nature (e.g. an antibody variable region), or alternatively may be designed *de novo* based on knowledge of the shape and/or charge of the antigen. Another possibility is that the receptor is based on a molecule identified by randomly screening a large number of candidate molecules.

In a preferred form of the invention the cell is engineered to express a chimeric receptor molecule incorporating an extracellular single-chain antibody domain (scFv) and a transmembrane and cytoplasmic signaling domain can specifically direct anti-tumor immune responses in a MHC-independent manner at targets normally capable of evading immune recognition. These targets also often lack important co-stimulatory ligands for maximal T cell activation. The therapeutic potential of engineered T cells extends from studies demonstrating specific antigen binding and target cell lysis in a range of different mouse tumor models (Haynes et al, 2001a; Haynes et al, 2001b; Kershaw et al, 2004; Darcy et al, 2000; Hwu et al, 1995) to their successful transfer into patients with minimal side effects in Phase I clinical trials (Mitsuyasu et al, 2000).

A number of recent studies have demonstrated that the most potent scFv receptor for maximal activation of both primary mouse and human T cells was a chimera incorporating both CD28 co-stimulatory and CD3 ζ signaling motifs linked in tandem in the intracellular domain (scFv-CD28-ζ) (Haynes et al, 2002a; Haynes et al, 2002b; Finney et al, 1998; Hombach et al, 2001b). These studies demonstrated that T cells engineered with this novel receptor could more effectively secrete cytokine and proliferate after antigen ligation compared with a chimera containing the ζ signaling domain alone (scFv-ζ) (Haynes et al, 2002a; Haynes et al, 2002b). In addition, studies in Applicants' laboratory have demonstrated that primary mouse T cells expressing the scFv-CD28-ζ chimera could more effectively eradicate established subcutaneous and metastatic disease in several different animal tumor models (Kershaw et al, 2004). Through mutation studies of the CD28 cytoplasmic signaling domain, the enhanced effector T cell function provided by the scFv-CD28-ζ receptor was found to be totally dependent on the CD28 co-stimulatory component (Moeller et al, 2004). Interestingly, the adoptively transferred T cells used in these experiments consistently comprised mainly CD8⁺ T cells and typically only a small number of CD4⁺ T cells (∼10%).

Preferably the molecule capable of binding to the antigen includes sequences from TCR-ζ and/or CD28 co-stimulatory signalling domains, or functional equivalents thereof. Adoptive immunotherapy strategies involving the genetic modification of autologous T cells with scFv chimeric receptors or TCR genes is gaining wider acceptance as a promising treatment for cancer. Indeed, recent advances in the design of scFv receptors comprising both primary TCR-ζ and CD28 co-stimulatory signaling domains have demonstrated striking results against early subcutaneous tumor growth and tumor metastases in mice after adoptive transfer (Haynes et al, 2002a; Haynes et al; 2002b).

As used herein the term "functional equivalent" is intended to mean any amino acid sequence having the same -or-similar biological -function to the TCR-ζ and CD28 co-stimulatory signalling domains expressly described herein. The skilled person will understand that various substitutions, insertions, deletions, and truncations may be made to an amino acid sequence while still retaining at least a proportion of the biological activity of the original protein. Furthermore, the use of amino acids other than natural amino acids may be used in the context of the present invention. Given the teachings of the instant specification, it will be possible for the skilled person to manipulate amino acid sequences described herein and test whether the adoptive immunotherapeutic response is effective. In some cases, the response will be the same or similar, in other cases the response will be less efficient, and in other cases the response will be more efficient.

One embodiment described herein uses scFv-engineered T cells incorporating inbuilt 'co-stimulatory' activity, that are able to recognize tumor targets in a non-MHC restricted manner clearly addressing the problems associated with MHC class I downregulation and lack of expression of critical co-stimulatory ligands by tumor cells. Despite this, these studies revealed that adoptive transfer of either scFv-CD28-ζ transduced CD8⁺ or CD4⁺ T cells alone had no anti-tumor effect which was consistent with a lack of persistence of these gene-engineered at the tumor site. In a very highly preferred form of the invention, the T cell is transduced with a construct as shown in Fig 7.

Preferably the T cell population is obtained from the subject, however it is contemplated that cells could be obtained from a twin or other family member, or from an appropriate tissue-matched unrelated donor. It may also be possible to use a continuous cell line.

The skilled person will understand that the present invention is essentially generic and will be useful for directing a cell mediated immune response against a broad range of antigens such as those found in or on tumor cells, or antigens found in or on cells infected with a virus (for example HIV), a Mycobacterium (e.g. *Mycobacterium tuberculosis*) or a parasite (e.g. *Plasmodium falciparum*)*.* However, in a preferred embodiment of the method the antigen is an antigen associated with a cancerous cell. Preferably the antigen is mutant oncogene protein or a tumor-associated cell surface protein. Preferably antigen is selected from the group consisting of Le^{y}, EGFR, PMSA, G250, NY-ESO1, CD20, CD19, idiotype Ig, p53, ras, CEA, MUC1, GD2, and HuD. In a highly preferred form of the method the antigen is Le^{y} or erbB2.

The present method requires that the T cells are engineered to express a molecule capable of binding to the antigen. As used herein, the term "engineering" is intended to include any process by which a T cell can be genetically altered so that a molecule capable of binding to an antigen of interest is produced. The skilled person is familiar with a range of methods capable of genetically altering mammalian cells, including the use of particle bombardment, electroporation, and retrovirus vectors. However, an important issue for application of gene therapy in the clinic involving retroviral gene transfer is the potential risk of insertional oncogenesis. This risk was recently highlighted when two out of ten SCID-X1 patients transfused with gene-modified autologous bone marrow derived progenitor and stem cells developed T cell leukemia (Hacein-Bey-Abina et al, 2003). However, in the case of T-cell adoptive immunotherapy approaches there has been no report of T cell transformation in animal models or in patients (Haynes et al, 2002a; Haynes et al, 2002b; Mitsuyasu et al 2000; Yee et al, 2002; Rosenberg et al, 1986). Consistent with this no signs of transformation were observed in the Applicants' long term surviving mice even after tumor rechallenge despite the fact that persistence of the gene-modified cells could be detected in both the spleen and peripheral blood. In any case if a problem were to arise with the transfer of genetically modified T cells in patients, a suicide gene strategy involving hsv-tk (Cohen et al, 1999) or the cytoplasmic domain of Fas (Thomis et al, 2001) could be employed. Alternatively the design of new integration vectors that target sites of the genome that are considered "safe" could be tested in future trials.

Preferably the engineered cells persist locally at the tumor or infection site in the subject long term. In one form of the method the engineered cells persist at the tumour or infection site for at least 100 days. This was demonstrated by the fact that (a) T cells were found at the tumour site (these cells cannot have been endogenous since a scid mouse was used, (b) the T cells were identified by FACS analysis as having been engineered. The persistent cells were also functional since it has been shown that subjects remained tumour free for 100 days (see Fig 6A) One of the striking features of this study was the ability of the gene engineered CD8⁺ and CD4⁺ T cells to persist long term and specifically reject a subsequent tumor rechallenge. To the best of the Applicant's knowledge this has never been reported previously for adoptively transferred gene-engineered T cells and potentially represents a significant step for application of this type of therapy in the clinic for treatment of not only patients with primary tumors but in cases of tumor relapse where cancer deposits have metastasized to multiple sites.

In a preferred form of the method, the adoptive immune response leads to the complete eradication of the infection or tumor.

While a number of forms of administration of the cells will be acceptable, a preferred form of the method provides that administration is by systemic injection. The ability to administer the cells systemically reveals a further advantage of the present method in that the Applicants have demonstrated the ability of the cells to travel to and deposit at the site of infection or tumour or other pathology from a remote location. This is an important difference between the methods described herein and that of Gyobu et al 2004 who described anti-tumor responses following combined transfer of target cells along with transduced human CD8⁺ and CD4⁺ T cells. Thus, the anti-tumor effects in mice were only observed by Gyobu et al., following preincubation of effector and target cells prior to injection. This is very different to the methods described herein whereby the engineered T cells are administered systemically, with the cells trafficking to the tumor site. In contrast the Applicants have demonstrated complete eradication of established tumor at a distant site following systemic injection of transduced mouse CD8⁺ and CD4⁺ T cells. In addition, the studies demonstrate long-term persistence and function of these gene-engineered T cells after tumor rechallenge.

Another difference between the work of Gyobu et al and the methods disclosed herein is the inability of human IFN-γ released by transduced human T cells to interact with the endogenous host IFN-γ receptors. Indeed, this has been demonstrated in this study using donor T cells from IFN-γ^{-/-} mice a role for IFN-γ secreted by gene engineered mouse CD8⁺ T cells for anti-tumor rejection. However this effector arm and possibly important effects from other cytokines were absent in the study by Gyobu et al.

Preferably the adoptive immune response is capable of preventing establishment of a new tumor or infection. The experiments described herein demonstrate for the first time *in vivo* that the provision of engineered antigen-specific CD4⁺ T cells can significantly improve anti-tumor responses, maintain persistence of transferred T cells and induce a potent recall response after tumor rechallenge. This is a substantial advance over the adoptive immunotherapy methods of the prior art.

In another aspect the present invention provides a composition for adoptive immunotherapy including an engineered CD4+ T cell and an engineered CD8+ T cell.

Preferably, the effective number of engineered CD4+ cells is greater than a number selected from the group consisting of about 1 x 10⁶, about 2.5 x 10⁶, about 5 x 10⁶, about 7.5 x 10⁶, and about 9 x 10⁶. Where the composition is to be used with an adult human subject, between about 10⁸ and about 10¹² CD4+ cells may be used.

Preferably, the effective number of engineered CD8+ cells is greater than a number selected from the group consisting of about 1 x 10⁶, about 2.5 x 10⁶, about 5 x 10⁶, about 7.5 x 10⁶, and about 9 x 10⁶. Where the composition is to be used with an adult human subject, between about 10⁸ and about 10¹² CD8+ cells may be used.

In a highly preferred embodiment of the composition the effective number of engineered CD4+ cells is about 5 x 10⁶ and the effective number of engineered CD8⁺ cells is about 5 x 10⁶. Where the composition is for use with an adult human subject, about 10¹⁰ and 10¹¹ each of CD4+ and CD8+ cells may be used.

In another aspect the present invention provides a composition for adoptive immunotherapy consisting of an engineered CD4+ T cell and an engineered CD8+ T cell. Preferably, the effective number of engineered CD4+ cells is greater than a number selected from the group consisting of about 1 x 10⁶, about 2.5 x 10⁶, about 5 x 10⁶, about 7.5 x 10⁶, and about 9 x 10⁶. Where the subject is an adult human, between about 10⁸ and about 10¹² CD4+ cells may be used. Applicants have surprisingly found that it is not necessary to include a target cell in the composition before administration to the subject. This is of considerable advantage since it is not necessary to remove tissue containing a target cell from the subject. For example, the clinician may know that the subject has a particular tumour (for example by the appearance of a tumour associated antigen in the serum), but is not required by the present invention to remove tumour tissue from the subject. Instead, the clinician could administer CD4+ and CD8+ cells capable of binding to that specific type of tumour.

Preferably, the effective number of engineered CD8+ cells is greater than a number selected from the group consisting of about 1 x 10⁶, about 2.5 x 10⁶, about 5 x 10⁶, about 7.5 x 10⁶, and about 9 x 10⁶. Where the subject is an adult human, between about 10⁸ and about 10¹² CD8+ cells may be used.

In a highly preferred embodiment of the method the effective number of engineered CD4+ cells is about 5 x 10⁶ and the effective number of engineered CD8+ cells is about 5 x 10⁶. Where the subject is an adult human, between about 10¹⁰ and 10¹¹ each of CD4+ and CD8+ cells may be used.

In yet a further aspect the present disclosure provides a method for treating or preventing a cancer or an infection, said method including administering to a animal in need thereof an engineered CD4+ T cell and an engineered CD8+ T cell wherein the CD4+ T cell and CD8+ T cell are engineered to express a molecule capable of binding to an antigen of the cancer or infection. It should be understood that the method is essentially generic in nature and may be applied to many forms of cancer or infection. The skilled person will understand that to attack a particular target cell, it would be necessary to first identify an antigen that is essentially unique to that cell, and typically not found on the cells of healthy tissue, or at least not found in any appreciable amounts. Once the antigen is identified, a molecule capable of binding to the antigen is identified. The CD4+ and CD8+ cells are then genetically altered to express the molecule capable of binding to the antigen, possibly with other co-stimulatory factors as described herein. By routine experimentation, the skilled person could then identify optimal means for administering the cells to the subject. However, a particular advantage of the method is that the engineered cells persist for extended periods at the infection or tumour site. Accordingly, the method is preferably used in respect of particularly refractory cancers such as breast cancer, colon cancer, or ovarian cancer. However, this does not limit the broad applicability of the method to treat any other cancer. Indeed, some tumor-associated antigens (such as Le^{y}) are found in a range of different cancers, and accordingly the present invention could be used to treat two or more cancer types in the same or different patients. The cancer may be present as a primary tumour or a metastasis from a primary tumour.

In yet a further aspect the present disclosure provides a method for treating or preventing a cancer or an infection, said method including administering to a animal in need thereof a composition described herein.

In yet a further aspect the present disclosure provides a method for treating or preventing a cancer or an infection, said method including a method for producing an adoptive immune response described herein, or a composition as described herein. The method may be used in respect of any cancer, however cancers including cells displaying the marker erbB2 are preferably involved. In one embodiment of the method, the treatment or prevention is for relapse of a cancer.

The methods of treatment or prevention may be used in combination with means for lowering the immunocompetence of the subject. Preferably the means is a non-myeloablative, for example by administration of cyclophosphamide. In this study scid mice were used for T cell transfer experiments due to the immunogenic nature of the human erbB2 target antigen. It would be expected that gene-engineered T cells can persist and induce potent recall responses in an immunocompetent host, such as a patient. The experiments described here using the scid system are relevant and could be mimicked in patients by a non-myeloablative regimen involving cyclophosphamide administration. Such regimens are currently being used in patients receiving melanoma-specific T cells which may enhance the therapeutic effect by dampening the immunosuppressive effect of T regulatory cells (Dudley et al, 2002).

Also provided by the present invention is the use of a composition as described herein in the manufacture of a medicament for the treatment or prevention of a cancer.. In one form of the invention, the cancer includes a cell displaying the marker erbB2.

A further aspect of the present disclosure provides a method of delivering an engineered CD4+ T cell and an engineered CD8+ T cell to a target cell in a subject, said method including the steps of administering the engineered CD4+ T cell and the engineered CD8+ T cell to the subject at a site remote from the target cell, allowing the engineered CD4+ T cell and the engineered CD8+ T cell to traffic to the target cell, and allowing the engineered CD4+ T cell, engineered CD8+ T cell and the target cell interact to provide an immune response against the target cell.

In another aspect the present invention provides a method of producing a cell population for adoptive immunotherapy against an antigen, said method including the steps of providing a population of CD4+ and CD8+ T cells from an immune tissue from a subject, increasing the number of CD4+ cells, or increasing the ratio of CD4+ T cells to CD8+ T cells in the population, and engineering the CD4+ and CD8+ T cells to express a molecule capable of binding to the antigen.

As will be understood by the skilled person, the steps of increasing the ratio and the engineering make take place in any order. However, preferably the step of increasing the number precedes that of engineering the cells.

Preferably the immune tissue is selected from the group consisting of the spleen, thymus, lymph node, bone marrow, or the blood may be used. Alternatively, tumour or infected tissue could be used as these tissues will contain CD4+ and CD8+ cells useful in the context of the present invention. For human therapy it is contemplated that blood or tumour is the more appropriate source.

Preferably, the effective number of engineered CD4+ cells is greater than a number selected from the group consisting of about 1 x 10⁶, about 2.5 x 10⁶, about 5 x 10⁶, about 7.5 x 10⁶, and about 9 x 10⁶. Where the subject is an adult human, between about 10⁸ and about 10¹² CD4+ cells may be used.

Preferably, the effective number of engineered CD8+ cells is greater than a number selected from the group consisting of about 1 x 10⁶, about 2.5 x 10⁶, about 5 x 10⁶, about 7.5 x 10⁶, and about 9 x 10⁶. Where the subject is an adult human, between about 10⁸ and about 10¹² CD8+ cells may be used.

In a highly preferred embodiment of the method the effective number of engineered CD4+ cells is about 5 x 10⁶ and the effective number of engineered CD8+ cells is about 5 x 10⁶. Where the subject is an adult human, between about 10¹⁰ and 10¹¹ each of CD4+ and CD8+ cells may be used.

The present invention will now be more fully described in the following non-limiting Examples.

### EXAMPLES

### EXAMPLE 1: Materials and Methods

The following materials and methods were implemented for experiments described in Examples 2 to 6.

*Cell Culture.* MC38 mouse B6 colon adenocarcinoma cells, the erbB2-expressing MC38-erbB2 cells, MDA-MB-435 human mammary carcinoma cells, the erbB2-expressing MDA-MB-435 cells, 4T1.2 mouse mammary carcinoma cells and the erbB2-expressing 4T1.2-erbB2 cells were cultured in DMEM supplemented with 10% (v/v) FCS, 2 mM glutamine, 100 U/ml penicillin and 100 µg/ml streptomycin (Life Technologies, Grand Island, NY). These tumor cell lines were CD80⁻ and CD86⁻. The ecotrophic GP+E86 retroviral packaging cells and the amphotrophic PA317 packaging cells were cultured in supplemented DMEM. Retrovirus-producing GP+E86 cells were cultured in DMEM containing 0.5 mg/ml G418 (Life Technologies). Retrovirally transduced splenic T cells were cultured in DMEM with 100 U/ml of human rIL-2 (kindly provided by Chiron, Emeryville, CA).

*Mice.* BALB/c and BALB/c *scid*/*scid* (scid) mice were purchased from The Walter and Eliza Hall Institute of Medical Research, Melbourne, Australia. BALB/c IFN-γ -deficient (BALB/c IFN-γ^{-/-}) mice were bred at the Peter MacCallum Cancer Centre, Melbourne. Mice of 6 to 12 weeks of age were used in experiments that were performed in accordance with animal experimental ethics committee guidelines.

*Generation of scFv-receptor transduced CD8*⁺ *and CD4*⁺ *mouse T cells.* A chimeric gene construct composed of the scFv-anti-erbB2 monoclonal antibody (mAb), a membrane proximal hinge region of human CD8, and the transmembrane and cytoplasmic regions of the mouse CD28 signaling chain fused to the cytoplasmic region of human TCR-ζ (scFv-anti-erbB2-CD28-ζ) was cloned into the retroviral vector pLXSN as previously described (Haynes et al, 2002b). A stable GP+E86 ecotrophic packaging cell line expressing the scFv-anti-erbB2-CD28-ζ receptor was isolated as previously described (Haynes et al, 2002b). Transduction of mouse splenic T lymphocytes was performed using GP+E86 clones producing ∼10⁷ cfu/ml. Briefly, mouse BALB/c donor spleen cells were depleted of red blood cells (RBCs) by hypotonic lysis with NH₄Cl and passed through a nylon wool syringe for enrichment, as described previously (Darcy et al, 2000; Haynes et al, 2001). To generate transduced CD8⁺ and CD4⁺ T cells each T cell subset was initially isolated by labelling with anti-CD4 or anti-CD8- magnetic beads (Miltenyi Biotec) and passed through a MACS depletion column. The efficiency of isolating separate T cell subsets was verified by flow cytometry. Enriched CD8⁺, CD4⁺ or unfractionated T cell cultures (10⁷ cells) were immediately co-cultured with retrovirus-producing packaging cells (5 x 10⁵) for 72 h in DMEM supplemented with with 4 µg/ml polybrene, 5 µg/ml PHA (Sigma, St Louis, MO) and 100 U/ml rIL-2. Following co-culture, T cells were analysed for transduction efficiency by flow cytometry.

*Flow Cytometry.* Expression of the α-erbB2-CD28-ζ chimeric receptor on the surface of CD8⁺, CD4⁺ or unfractionated transduced mouse T cells was determined by indirect immunofluorescence with a *c-myc* tag Ab, followed by staining with a PE-labeled anti-mouse Ig mAb (BD Biosciences, San Jose, CA). Background fluorescence was assessed using the PE-labeled anti-mouse Ig mAb alone. Cell surface phenotyping of transduced cells was determined by direct staining with FITC-labeled anti-CD4 (RM4-5; PharMingen) and PE-labeled anti-CD8 (53-6.7; PharMingen) mAbs, as previously described. The unfractionated T cell population consisted of 80-85% CD8⁺ and 10-15% CD4⁺ T cells as previously demonstrated (Haynes et al, 2002a; Haynes et al, 2002b; Darcy et al 2000; Haynes et al, 2001).

*Antigen-specific cytotoxicity, cytokine secretion and proliferation by transduced T cells.* The ability of transduced T cells to specifically mediate target cell lysis was assessed in a 6 h chromium release assay, as described previously (Darcy et al 1998). The capacity of transduced T cells to produce cytokine after erbB2 antigen ligation was determined by enzyme-linked immunosorbent assay (ELISA) (PharMingen). Mouse CD8⁺, CD4⁺ or unfractionated T cells (10⁶) transduced with the scFv-α-erbB2-CD28-ζ receptor or mock CD8⁺ and CD4⁺ T cells transduced with an empty LXSN vector were co-cultured with 10⁶ erbB2⁺ MC-38-erbB2, MDA-MB-435-erbB2 or 4T1.2-erbB2 tumor cells or erbB2⁻ MC-38, MDA-MB-435 or 4T1.2 parental tumor cells in 12-well plates for 20 h. No exogenous IL-2 was added. Supernatants were harvested and IFN-γ, IL-2, GM-CSF, and IL-4 production by transduced T cells was determined by ELISA. Cytokine released through stimulation of endogenous CD3 and CD28 receptors was assessed for each T-effector cell population using plate-bound CD3 (1 µg/mL) (145.2C11; PharMingen) and CD28 (1µg/mL) (37.51; PharMingen) mAbs. The proliferative capacity of transduced T cells was assessed in a [³H]-thymidine incorporation assay. Engineered mouse T cells (10⁵ cells/ml) were cultured in 96-well U bottom plates and stimulated with plate bound anti-tag mAb or CD3 plus CD28 control mAb (1 µg/ml) for 3 days. The cultures were then pulsed with 0.5 µCi/well (0.0185 MBq) of [³H]-thymidine (Amersham, Aylesbury, United Kingdom) for the final 16 h of the culture period and the incorporation of radioactivity was measured in a TRI-CARB 2100TR Liquid Scintillation Counter (Packard, Meriden, CT).

*Immunohistochemistry.* Hematoxylin/Eosin staining and immunohistochemistry were performed on frozen sections. Antibodies used were FITC-anti-mouse CD4, biotin-anti-mouse CD8b.2, APC-anti-mouse CD11b, FITC-anti-mouse CD11b (all from Becton Dickenson), biotin-anti-myc tag (Abcam), isotype controls and a streptavidin Alexa® 594 secondary (Molecular Probes).

*Polymerase chain reaction (PCR).* The detection of gene-engineered T cells following adoptive transfer in mice was assessed by PCR amplification of the neomycin phosphotransferase gene. Mice bearing 5-day MDA-MB-435 tumor were injected with scFv-CD28-ζ transduced CD8⁺ and CD4⁺ T cells and were eye bled or spleens removed at various time points. Red blood cells were lysed using ACK lysis buffer (RT for 5 min.), washed, and cells resuspended at 5x10⁶ cells/200µl. Genomic DNA from peripheral blood or splenocytes was subsequently purified for PCR using QIAamp® DNA Blood MiniKit as per manufacturer's instructions (QIAGEN, Clifton Hill, Australia). Neomycin sense primer was 5'-TGGCTGCTATTGGGCGAAGT-3'; antisense was 5'-TATCACGGGTAGCCAACGCT-3', mouse β-actin sense primer was 5'-AGGCGGTGCTGTCCTTGTAT-3'; antisense was 5'-GGAAGGAAGGCTGGAAGAGT-3'. The forward and reverse primers for both neomycin and β-actin genes were designed to amplify fragments of approximately 400 bp using Platinum Pfx DNA polymerase (Invitrogen Life Sciences, Carlsbad, CA, USA). Conditions for PCR amplification of neomycin and β-actin DNA were 30 cycles, consisting of denaturation at 95°C for 30 s, annealing at 58°C for 30 s, and extension at 68°C for 1.5 min. Amplified products were quantitated using 1 kb Plus markers (invitrogen Life Sciences, Carlsbad, CA, USA) on a 1% w/v agarose gel and visualized with ethidium bromide fluorescence.

*Adoptive Transfer Model.* The anti-tumor activity of transduced T cells was assessed against the MDA-MB-435-erbB2 tumor cell line as previously described (Haynes et al, 2002b). Briefly, scid mice were injected i.v. with 5 x 10⁶ human MDA-MB-435-erbB2 breast carcinoma cells to establish pulmonary metastases. Unfractionated scFv-CD28-ζ transduced T cells (10⁷), transduced CD8⁺ or CD4⁺ T cells alone (10⁷), or a 1:1 combination of transduced CD8⁺ and CD4⁺ T cells (5 x 10⁶ of each) from BALB/c donor mice were injected i.v. into groups of 5-10 mice at days 5 or 10 after tumor inoculation. For experiments evaluating minimum number of engineered CD4 required to achieve total tumor regression, mice received different ratios of transduced CD8⁺ and CD4⁺ T cells. The adoptive transfer of T cells transduced with an irrelevant scFv receptor (scFv-α-CEA-γ) or mock transduced with an empty vector pLXSN served as controls. In addition, adoptive transfer of scFv-transduced CD8⁺ and CD4⁺ T lymphocytes (5 x 10⁶ of each, day 5) from either BALB/c-wildtype or BALB/c IFN-γ^{-/-} donor mice were used to evaluate the role of IFN-γ released by either T cell subsetin the anti-tumor effect. All mice were monitored daily for tumor growth, indicated by respiratory distress and loss of body condition. Tumor growth was assessed as follows; (Haynes et al, 2002a) in survival experiments, mice that were morbid were sacrificed and the day of death and lung weights recorded (Haynes et al, 2002b) or mice were sacrificed at days 6, 12 or 16 and harvested lungs were fixed in 10% formalin, embedded, sectioned and stained with H & E for histological examination or frozen sections of lungs were made for immunohistochemical analysis.

*Tumor rechallenge experiments.* Mice long term surviving the primary MDA-MB-435 tumor (>100 days) were rechallenged with an i.v. injection of 5 x 10⁶ human MDA-MB-435-erbB2 tumor cells and survival monitored over 100 days. Injection of 5 x 10⁶ MDA-MB-435-erbB2 tumor cells into normal scid mice served as a control. In another set of experiments mice were rechallenged with a subcutaneous injection of the mouse mammary carcinoma 4T1.2-erbB2 cells or 4T1.2 parental cells at 5 x 10⁴ (high dose) or 5 x 10³ (low dose). Survival of mice was monitored daily and defined as the period with no overt signs of distress (lethargy, labored breathing, ruffled or huddled appearance), as assessed by two independent observers. Subcutaneous tumor growth was measured by a caliper square along the perpendicular axes of the tumors. Data were recorded as either percentage survival or the mean tumor size (mm², product of the two perpendicular diameters) ± SEM. Injection of 4T1.2-erbB2 cells and 4T1.2 parental tumor cells into normal scid mice at high and low dose were included as controls in these experiments.

### EXAMPLE 2: Expression of the scFv-CD28-ζ receptor in transduced CD8⁺ and CD4⁺ primary mouse T cells.

Consistent with Applicants' studies, data disclosed herein demonstrates that retroviral transduction of unfractionated PHA/IL-2 stimulated splenic T cells consistently resulted in a high proportion of CD8⁺ T cells (80-85%) but low numbers of CD4⁺ T cells (10-15%) (Fig. 1 A and B) (Haynes et al, 2002a; Haynes et al, 2002b). To determine whether expression of the chimeric anti-erbB2-CD28-ζ receptor could be achieved in a greater percentage of CD4⁺ T cells, CD8⁺ and CD4⁺ T cells were isolated by magnetic bead depletion prior to transduction. Using this approach, it was possible to achieve good levels of expression of the chimeric anti-erbB2-CD28-ζ receptor in CD8⁺ and CD4⁺ T cells after staining with a c-myc anti-tag antibody (Fig. 1 D and F) compared to mock transduced CD8⁺ or CD4⁺ T cells (Fig. 1 H and J). Importantly, the T cell populations consisted of greater than 95% CD8⁺ or CD4⁺ T cells following transduction (Fig. 1 C,E,G and I). There was negligible expression of the chimeric receptor in either the CD8⁻ or CD4⁻ populations (data not shown).

### EXAMPLE 3: Transduced CD8⁺ and CD4⁺ mouse T cells mediate antigen-specific cytokine secretion, proliferation and tumor cell lysis.

The functional capacity of either unfractionated, CD4⁺ or CD8⁺ transduced T cells was compared in a number of different in vitro assays. Applicants have previously shown the ability of scFv-CD28-ζ unfractionated engineered T cells to secrete Tc1 cytokines (IFN-γ and GM-CSF) upon antigen stimulation with erbB2⁺ target cells (Haynes et al, 2002b; Kershaw et al, 2004). Thus, it was of interest-to determine the cytokine secretion pattern of isolated CD8⁺ and CD4⁺ T cell populations transduced with the scFv-CD28-ζ receptor. Following co-culture with MDA-MB-435-erbB2 tumor cells, transduced CD8⁺ T cells produced high levels of Tc1 cytokines IFN-γ and GM-CSF (Fig. 2 A and B). However, transduced CD4⁺ T cells produced high levels of both Tc1 (IFN-γ, GM-CSF, IL-2) (Fig. 2 A, B and D) and Tc2 (IL-4) (Fig. 2 C) cytokines. Consistent with previous studies, unfractionated transduced T cells produced high levels of IFN-γ and GM-CSF but no IL-4 or IL-2 cytokines (Fig. 2 A-D). The secretion of cytokines by scFv-CD28-ζ transduced CD8⁺ and CD4⁺ was antigen-specific since transduced T cells co-cultured with erbB2⁻ MDA-MB-435 parental tumor cells demonstrated no significant cytokine secretion. In addition, mock transduced CD8⁺ and CD4⁺ co-cultured with either cell line secreted no measurable cytokine. Comparative results were also demonstrated against the mouse colon adenocarcinoma cell line MC38-erbB2 or mouse mammary carcinoma cell line 4T1.2-erbB2 (data not shown). The proliferative capacity of the various T cell populations transduced with the scFv-CD28-ζ receptor were next examined by measuring [³H]-thymidine incorporation following stimulation with plate bound anti-tag antibody. Interestingly, there was no significant difference in the level of proliferation observed by transduced CD4⁺, CD8⁺ or unfractionated T cells following receptor ligation and proliferation by all transduced T cell populations was comparable to stimulation through the endogenous T cell and CD28 receptors (Fig. 2 E). No proliferation was observed by mock transduced CD4⁺, CD8⁺ or unfractionated T cells following stimulation with anti-tag mAb (Fig. 2 E). Lastly, the ability of unfractionated, CD4⁺ and CD8⁺ T cells engineered with the scFv-CD28-ζ receptor to mediate specific lysis of MDA-MB-435-erbB2 target cells in a 6 hr ⁵¹Cr release assay was compared. Interestingly, transduced unfractionated and CD8⁺ T cells mediated greater cytolytic activity of MDA-MB-435-erbB2 target cells compared with transduced CD4⁺ T cells (Fig. 2 F). No lysis of erbB2⁻ MDA-MB-435 parental tumor cells was observed by any of the scFv-CD28-ζ engineered T cell populations and mock transduced CD8⁺ and CD4⁺ T cells could not lyse either cell line (Fig. 2 G). Taken together, these data demonstrated that both CD8⁺ and CD4⁺ isolated T cells could be effectively transduced with the scFv-CD28-ζ receptor and mediate antigen-specific cytokine release, proliferation and cytolytic activity.

### EXAMPLE 4: Increased transfer of engineered CD4⁺ T cells enhances tumor free survival of mice.

The adoptive transfer of unfractionated scFv-α-erbB2-CD28-ζ engineered T cells containing ∼10% transduced CD4⁺ T cells into scid mice bearing 5 day MDA-MB-435-erbB2 lung metastases has previously demonstrated between 20-50% long-term survival of mice (Haynes et al, 2002b). To test whether increasing the proportion of gene-modified CD4⁺ T cells could enhance the anti-tumor response in this model, survival of mice treated with unfractionated scFv-CD28-ζ transduced T cells (10⁷) or the same total number of CD8⁺ and CD4⁺ T cells (5 x 10⁶ cells of each) were compared. Strikingly, 100% of mice bearing 5 day MDA-MB-435-erbB2 lung metastases that received a 1:1 ratio of scFv-CD28-ζ-engineered CD8⁺ and CD4⁺ T cells survived compared to 30% survival of mice treated with unfractionated transduced T cells (*p*< 0.005) (Fig. 3 A). The enhanced anti-tumor response was entirely dependent on the transfer of appropriately targeted CD4⁺ T cells, since mice treated with a 1:1 combination of transduced CD8⁺ T cells and naïve CD4⁺ T cells or transduced CD8⁺ T cells and CD4⁺ T cells engineered with an irrelevant scFv-α-CEA-γ receptor did not survive beyond 20 days (Fig. 3 B). Interestingly, no anti-tumor effect was observed in mice that received either transduced CD4⁺ or CD8⁺ T cells alone indicating a requirement for co-operation of the two types of T cell (Fig. 3 A). In addition, mice receiving a 1:1 ratio of transduced CD8⁺ and CD4⁺ T cells of irrelevant specificity or no T cells did not survive (Fig. 3 A).

### EXAMPLE 5: Detection of transduced CD8⁺ and CD4⁺ T cells in vivo.

Given the striking response of adoptively transferred engineered CD8⁺ and CD4⁺ T cells against established lung metastases Applicants investigated whether transduced T cells could be detected at the tumor site. Hematoxylin and Eotaxin (H & E) staining of mouse lung sections 16 days after tumor inoculation demonstrated significant reduction in lung tumor burden in mice treated with 1:1 transfer of scFv-α-erbB2-CD28-ζ engineered CD8⁺ and CD4⁺ T cells compared to control T cells (Fig. 5 A and D). In contrast, the number of mice responding after transfer of transduced unfractionated T cells was significantly reduced as demonstrated by two representative H&E lung sections at day 16 (Fig. 5 B and C). Confocal microscopy following immunohistochemical staining demonstrated presence of both CD8⁺ and CD4⁺ T cells in day 16 lung sections of mice treated with 1:1 transfer of transduced CD8⁺ and CD4⁺ T cells (Fig. 5 E). The detection of D-tag staining in lung sections of treated mice suggested that these T cells were expressing the chimeric scFv-CD28-ζ receptor (Fig. 5 I). As demonstrated previously (Kershaw et al, 2004), only CD8⁺ T cells and □-tag positive staining were detected in lungs of mice that responded to treatment with unfractionated T cells (Fig. 5 F and J). No staining was observed in mice that did not respond to treatment with unfractionated T cell transfer (Fig. 5 G and K). In addition, no detection of control CD8⁺ and CD4⁺ T cells was observed in lung sections from mice (Fig. 5 H and L). Interestingly, neither transduced CD8⁺ or CD4⁺ T cells injected alone were detected in d16 lung sections which was consistent with no anti-tumor effect observed in these mice (data not shown). As controls, both CD8⁺ and CD4⁺ T cells were detected in spleens of BALB/c mice using the same immunohistochemical staining procedure, but not in lungs from normal scid mice (data not shown).

To further evaluate persistence of transduced T cells in long term surviving mice PCR amplification of the neomycin gene was performed. The presence of a ∼400 bp fragment corresponding to the neomycin gene in both peripheral blood and spleens of mice surviving tumor for >100 days after treatment with transduced CD8⁺ and CD4⁺ T cells indicated that these T cells could persist long term even after tumor eradication (Fig. 5 M). The neomycin gene was not detected in peripheral blood and spleens of normal BALB/c or scid mice (Fig. 5 M). The persistence of these T cells was further supported by flow cytometry which demonstrated the presence of both CD8⁺ and CD4⁺ T cells in peripheral blood of long- term surviving mice (data not shown). Overall, this data strongly suggested that the anti-tumor effect in mice was dependent on the localization at the site of tumor and long term persistence of adoptively transferred engineered CD8⁺ and CD4⁺ T cells.

### EXAMPLE 6: Antigen-specific response to tumor rechallenge.

An important issue with -regard to any-cancer-therapy is whether the specific treatment can induce a secondary response to tumor relapse. To test whether the scFv approach described herein could effectively mediate a response to secondary tumor rechallenge, long term surviving mice (>100 days) initially treated with transduced CD8⁺ and CD4⁺ T cells were injected with an i.v. dose of 5x10⁶ MDA-MB-435-erbB2 tumor cells. Remarkably, all mice were able to totally eradicate a second dose of MDA-MB-435-erbB2 tumor (Fig. 6 A). Normal scid mice injected with MDA-MB-435-erbB2 cells did not survive (Fig. 6 A). As a further test of specificity, long term surviving mice were rechallenged with a subcutaneous dose (5x10⁴) of mouse mammary carcinoma tumor cells expressing the human erbB2 antigen (4T1.2-erbB2) or 4T1.2 parental cells. As demonstrated above, all mice could significantly inhibit the s.c. growth of 4T1.2-erbB2 tumor compared to 4T1.2 parental tumor cells and their survival was significantly prolonged (Fig. 6 B and C). The s.c growth of 4T1.2-erbB2 or 4T1.2 parental tumor cells in normal scid mice was not comparatively inhibited (Fig. 6 B and C). To determine whether long-term surviving mice could totally eradicate a lower dose of 4T1.2-erbB2 tumor, mice were rechallenged with 5x10³ 4T1.2-erbB2 tumor cells. Strikingly, the s.c growth of 4T1.2-erbB2 at this lower dose was totally inhibited compared to s.c injection of 5x10⁴ 4T1.2-erbB2 tumor cells (Fig. 6 D). The s.c. growth of 4T1.2-erbB2 or 4T1.2 parental tumor cells at both high and low doses was not affected in control scid mice (Fig. 6 D). These results have clearly demonstrated for the first time that mice treated with gene-engineered T cells could mount a sustained and potent antigen-specific secondary response to tumor rechallenge.

### EXAMPLE 7: Adoptive immunotherapy of a human.

Following is a non-limiting prophetic example describing one method of adoptive immunotherapy on a human subject.

At day 0, peripheral blood will be isolated from the subject by apherisis. Cells will be stimulated with OKT3 and IL-2 according to standard protocols known in the art.

At day 3, CD8 and CD4 T cell subsets will be produced by fluorescence activated cell sorting. The T cells will then be transduced with a retroviral vector to express chimeric scFv-CD28-ζ receptor.

At day 4, transduction will be repeated to increase the proportion of transduced T cells in each subset.

From day 5 to day 18, cells will be further cultured and expanded and maintained at 1-2 x 10⁶/ml, adding IL-2 every 2-3 days.

At day 18, cells will be stimulated with allogeneic PBMC, OKT3 and IL-2, then culture and expand (maintaining IL-2 treatment).

At day 36, about 10⁸ to 10¹² cells will be harvested and infused into a patient via the intravenous route.

The entire process from day 0 to day 36 may be repeated two to three times depending on the response of the tumour or infection. Furthermore, maintenance therapy on a semi-regular basis may be provided.

### REFERENCES

Cohen, J.L., O. Boyer, V. Thomas-Vaslin, and D. Klatzmann. 1999. Suicide gene mediated modulation of graft-versus-host disease. Leuk Lymphoma 34:473-480.
Darcy, P.K., M.H. Kershaw, J.A. Trapani, and M.J. Smyth. 1998. Expression in cytotoxic T lymphocytes of a single-chain anti-carcinoembryonic antigen antibody. Redirected Fas ligand-mediated lysis of colon carcinoma. Eur J Immunol 28:1663-1672.
Darcy, P.K., N.M. Haynes, M.B. Snook, J.A. Trapani, L. Cerruti, S.M. Jane, and M.J. Smyth. 2000. Redirected perforin-dependent lysis of colon carcinoma by ex vivo genetically engineered CTL. J Immunol 164:3705-3712.
Dudley, M.E., J. Wunderlich, M.I. Nishimura, D. Yu, J.C. Yang, S.L. Topalian, D.J. Schwartzentruber, P. Hwu, F.M. Marincola, R. Sherry, S.F. Leitman, and S.A. Rosenberg. 2001. Adoptive transfer of cloned melanoma-reactive T lymphocytes for the treatment of patients with metastatic melanoma. J Immunother 24:363-373.
Dudley, M.E., J.R. Wunderlich, P.F. Robbins, J.C. Yang, P. Hwu, D.J. Schwartzentruber, S.L. Topalian, R. Sherry, N.P. Restifo, A.M. Hubicki, M.R. Robinson, M. Raffeld, P. Duray, C.A. Seipp, L. Rogers-Freezer, K.E. Morton, S.A. Mavroukakis, D.E. White, and S.A. Rosenberg. 2002. Cancer regression and autoimmunity in patients after clonal repopulation with antitumor lymphocytes. Science 298:850-854.
Finney, H.M., A.D. Lawson, C.R. Bebbington, and A.N. Weir. 1998. Chimeric receptors providing both primary and costimulatory signaling in T cells from a single gene-product. J Immunol 161:2791-2797
Finney, H.M., A.N. Akbar, and A.D. Lawson. 2004. Activation of resting human primary T cells with chimeric receptors: costimulation from CD28, inducible costimulator, CD134, and CD137 in series with signals from the TCR zeta chain. J Immunol 172:104-113.
Garrido, F., T. Cabrera, A. Concha, S. Glew, F. Ruiz-Cabello, and P.L. Stern. 1993. Natural history of HLA expression during tumor development. Immunol Today 14:491-499.
Gyobu, H., T. Tsuji, Y. Suzuki, T. Ohkuri, K. Chamoto, M. Kuroki, H. Miyoshi, Y. Kawarada, H. Katoh, T. Takeshima, and T. Nishimura. 2004. Generation and targeting of human tumor-specific Tc1 and Th1 cells transduced with a lentivirus containing a chimeric immunoglobulin T-cell receptor. Cancer Res 64:1490-1495.
Hacein-Bey-Abina, S., C. Von Kalle, M. Schmidt, M.P. McCormack, N. Wulffraat, P. Leboulch, A. Lim, C.S. Osborne, R. Pawliuk, E. Morillon, R. Sorensen, A. Forster, P. Fraser, J.I. Cohen, G. de Saint Basile, I. Alexander, U. Wintergerst, T. Frebourg, A. Aurias, D. Stoppa-Lyonnet, S. Romana, I. Radford-Weiss, F. Gross, F. Valensi, E. Delabesse, E. Macintyre, F. Sigaux, J. Soulier, L.E. Leiva, M. Wissler, C. Prinz, T.H. Rabbitts, F. Le Deist, A. Fischer, and M. Cavazzana-Calvo. 2003. LMO2-associated clonal T cell proliferation in two patients after gene therapy for SCID-X1. Science 302:415-419.
Haynes, N.M., M.B. Snook, J.A. Trapani, L. Cerruti, S.M. Jane, M.J. Smyth, and P.K. Darcy. 2001. Redirecting mouse CTL against colon carcinoma: superior signaling efficacy of single-chain variable domain chimeras containing TCR-zeta vs Fc epsilon RI-gamma. J Immunol 166:182-187.
Haynes, N.M., J.A. Trapani, M.W. Teng, J.T. Jackson, L. Cerruti, S.M. Jane, M.H. Kershaw, M.J. Smyth, and P.K. Darcy. 2002a. Rejection of Syngeneic Colon Carcinoma-by CTLs Expressing Single Chain Antibody Receptors Codelivering CD28 Costimulation. J Immunol 169:5780-5786.
Haynes, N.M., J.A. Trapani, M.W. Teng, J.T. Jackson, L. Cerruti, S.M. Jane, M.H. Kershaw, M.J. Smyth, and P.K. Darcy. 2002b. Single-chain antigen recognition receptors that costimulate potent rejection of established experimental tumors. Blood 100:3155-3163.
Hombach, A., C. Heuser, T. Marquardt, A. Wieczarkowiecz, V. Groneck, C. Pohl, and H. Abken. 2001a. CD4+ T cells engrafted with a recombinant immunoreceptor efficiently lyse target cells in a MHC antigen- and Fas-independent fashion. J Immunol 167:1090-1096.
Hombach, A., A. Wieczarkowiecz, T. Marquardt, C. Heuser, L. Usai, C. Pohl, B. Seliger, and H. Abken. 2001b. Tumor-specific T cell activation by recombinant immunoreceptors: CD3 zeta signaling and CD28 costimulation are simultaneously required for efficient IL-2 secretion and can be integrated into one combined CD28/CD3 zeta signaling receptor molecule. J Immunol 167:6123-6131.
Hwu, P., J.C. Yang, R. Cowherd, J. Treisman, G.E. Shafer, Z. Eshhar, and S.A. Rosenberg. 1995. In vivo antitumor activity of T cells redirected with chimeric antibody/T-cell receptor genes. Cancer Res 55:3369-3373.
Kershaw, M.H., J.T. Jackson, N.M. Haynes, M.W. Teng, M. Moeller, Y. Hayakawa, S.E. Street, R. Cameron, J.E. Tanner, J.A. Trapani, M.J. Smyth, and P.K. Darcy. 2004. Gene-engineered T cells as a superior adjuvant therapy for metastatic cancer. J Immunol 173:2143-2150.
Mitsuyasu, R.T., P.A. Anton, S.G. Deeks, D.T. Scadden, E. Connick, M.T. Downs, A. Bakker, M.R. Roberts, C.H. June, S. Jalali, A.A. Lin, R. Pennathur-Das, and K.M. Hege. 2000. Prolonged survival and tissue trafficking following adoptive transfer of CD4zeta gene-modified autologous CD4(+) and CD8(+) T cells in human-immunodeficiency-virus-infected subjects. Blood 96:785-793.
Moeller, M., N.M. Haynes, J.A. Trapani, M.W. Teng, J.T. Jackson, J.E. Tanner, L. Cerutti, S.M. Jane, M.H. Kershaw, M.J. Smyth, and P.K. Darcy. 2004. A functional role for CD28 costimulation in tumor recognition by single-chain receptor-modified T cells. Cancer Gene Ther 11:371-379.
Shimizu, J., S. Yamazaki, and S. Sakaguchi. 1999. Induction of tumor immunity by removing CD25+CD4+ T cells: a common basis between tumor immunity and autoimmunity. J Immunol 163:5211-5218.
Steitz, J., J. Bruck, J. Lenz, J. Knop, and T. Tuting. 2001. Depletion of CD25(+) CD4(+) T cells and treatment with tyrosinase-related protein 2-transduced dendritic cells enhance the interferon alpha-induced, CD8(+) T-cell-dependent immune defense of B16 melanoma. Cancer Res 61:8643-8646.
Taniguchi, T., and Y. Minami. 1993. The IL-2/IL-2 receptor system: a current overview. Cell 73:5-8.
Teng, M.W., M.H. Kershaw, M. Moeller, M.J. Smyth, and P.K. Darcy. 2004. Immunotherapy of cancer using systemically delivered gene-modified human T lymphocytes. Hum Gene Ther 15:699-708.
Theze, J., P.M. Alzari, and J. Bertoglio. 1996. Interleukin 2 and its receptors: recent advances and new immunological functions. Immunol Today 17:481-486.
Rosenberg, S.A., P. Spiess, and R. Lafreniere. 1986. A new approach to the adoptive immunotherapy of cancer with tumor-infiltrating lymphocytes. Science 233:1318-1321.
Thomis, D.C., S. Marktel, C. Bonini, C. Traversari, M. Gilman, C. Bordignon, and T. Clackson. 2001. A Fas-based suicide switch in human T cells for the treatment of graft-versus-host disease. Blood 97:1249-1257.
Yee, C., J.A. Thompson, D. Byrd, S.R. Riddell, P. Roche, E. Celis, and P.D. Greenberg. 2002. Adoptive T cell therapy using antigen-specific CD8+ T cell clones for the treatment of patients with metastatic melanoma: in vivo persistence, migration, and antitumor effect of transferred T cells. Proc Natl Acad Sci U S A 99:16168-16173.

## Claims

1. A composition for use in a method of treatment or prevention of cancer in a subject, the method comprising systemic injection of the composition, the composition comprising cells, wherein the cells consist of (a) a population of CD4+ T cells, the population comprising CD4+ T cells engineered to express a molecule capable of binding to an antigen on a target cell of the cancer; and (b) a population of CD8+ T cells, the population comprising CD8+ T cells engineered to express the molecule capable of binding to the antigen on the target cell of the cancer; wherein the ratio of engineered CD4+ T cells to engineered CD8+ T cells in the composition is greater than 1:3 and less than 3:1.

2. A composition for use in a method of treatment or prevention of cancer according to Claim 1; wherein the ratio of CD4+ T cells to CD8+ T cells in the composition is greater than 1:3 and less than 3:1.

3. A composition for use in a method of treatment or prevention of cancer according to claim 1 or claim 2 wherein the number of engineered CD4+ T cells in the composition is greater than a number selected from the group consisting of 1 x 10⁶, 2.5 x 10⁶, 5 x 10⁶, 7.5 x 10⁶, and 9 x 10⁶, and wherein the number of engineered CD8+ T cells in the composition is greater than a number selected from the group consisting of 1 x 10⁶, 2.5 x 10⁶, 5 x 10⁶, 7.5 x 10⁶, and 9 x 10⁶.

4. A composition for use in a method of treatment or prevention of cancer according to any of claims 1 to 3, wherein the number of engineered CD4+ T cells in the composition is between 10⁸ and 10¹² and wherein the number of engineered CD8+ T cells in the composition is between 10⁸ and 10¹².

5. A composition for use in a method of treatment or prevention of cancer according to any of claims 1 to 4, wherein each of the number of engineered CD4+ T cells and the number of engineered CD8+ T cells is between 10¹⁰ and 10¹¹.

6. A composition for use in a method of treatment or prevention of cancer according to any of claims 1 to 5, wherein the ratio of engineered CD4+ T cells to engineered CD8+ T cells in the composition is 1:1.

7. A composition for use in a method of treatment or prevention of cancer according to any of claims 1 to 6, wherein the molecule capable of binding to the antigen is encoded by a nucleotide sequence comprising a sequence encoding an amino acid sequence derived from TCR-ζ and/or comprising a sequence encoding an amino acid sequence derived from the CD28 co-stimulatory signalling domain.

8. A composition for use in a method of treatment or prevention of cancer according to any of claims 1 to 7, wherein the molecule is encoded by a chimeric gene construct encoding a single chain variable fragment of an antibody (scFv), a transmembrane and cytoplasmic region of a CD28 signalling chain and a cytoplasmic region of a T cell receptor, and wherein the scFv is capable of binding the antigen on the target cell.

9. A composition for use in a method of treatment or prevention of cancer according to claim 8, wherein the T cell receptor is TCR-ζ.

10. A composition for use in a method of treatment or prevention of cancer according to claim 8 or claim 9, wherein the chimeric gene construct further encodes a membrane proximal hinge region of CD8.

11. A composition for use in a method of treatment or prevention of cancer according to any of claims 1 to 10, wherein the antigen is found in or on a tumour cell.

12. A composition for use in a method of treatment or prevention of cancer according to claim 11, wherein the antigen is selected from erbB2, Cd19, Le^{y}, EGFR, PMSA, G250, NY-ESO1, CD20, idiotype Ig, p53, ras, CEA, MUC1, GD2 and HuD.

13. A composition for use in a method of treatment or prevention of cancer according to claim any one of the preceding claims, wherein the treatment or prevention of the cancer is for relapse of the cancer.

14. A composition for use in a method of treatment or prevention of cancer according to any of claims 1 to 13, wherein cells in the composition are derived from an immune tissue or tumor tissue of the subject and the ratio of CD4+ T cells to CD8+ T cells in the composition is increased compared to the ratio of CD4+ T cells to CD8+ T cells in the immune tissue or tumor tissue of the subject.

15. A composition for use in a method of treatment or prevention of cancer according to any one of the preceding claims, wherein the immune tissue or tumor tissue is selected from the group consisting of blood, spleen, thymus, lymph node or bone marrow.

16. A composition for use in a method of treatment or prevention of cancer according to claim 15, wherein the tissue is blood or a tumor tissue.

17. Use of a composition for the manufacture of a medicament for the treatment or prevention of a cancer, the composition comprising cells, wherein the cells consist of (a) a population of CD4+ T cells, the population comprising CD4+ T cells engineered to express a molecule capable of binding to an antigen on a target cell of the cancer; and (b) a population of CD8+ T cells, the population comprising CD8+ T cells engineered to express the molecule capable of binding to the antigen on the target cell of the cancer; wherein the ratio of engineered CD4+ T cells to engineered CD8+ T cells in the composition is greater than 1:3 and less than 3:1, and wherein the treatment or prevention of cancer involves systemic injection of the composition.

18. Use according to claim 17, wherein cells in the composition are derived from an immune tissue or tumor tissue of the subject and the ratio of CD4+ T cells to CD8+ T cells in the composition is increased compared to the ratio of CD4+ T cells to CD8+ T cells in the immune tissue or tumor tissue of the subject.

19. Use according to claim 18, wherein the immune tissue or tumor tissue is selected from the group consisting of blood, spleen, thymus, lymph node or bone marrow.

20. Use according to any one of claims 17 to 19 wherein the tissue is blood or a tumor tissue.

## Patentansprüche

1. Zusammensetzung zur Verwendung in einem Verfahren zur Behandlung oder zur Prävention von Krebs bei einem Individuum, wobei das Verfahren die systemische Injektion der Zusammensetzung umfasst, wobei die Zusammensetzung Zellen umfasst, wobei die Zellen aus (a) einer Population von CD4+-T-Zellen bestehen, wobei die Population CD4+-T-Zellen umfasst, die gentechnisch verändert sind, um ein Molekül zu exprimieren, das in der Lage ist, an ein Antigen auf einer Zielzelle des Krebses zu binden; und (b) einer Population von CD8+-T-Zellen bestehen, wobei die Population CD8-T-Zellen umfasst, die gentechnisch verändert sind, um das Molekül zu exprimieren, das in der Lage ist, an das Antigen auf der Zielzelle des Krebses zu binden; wobei das Verhältnis der gentechnisch veränderten CD4+-T-Zellen zu den gentechnisch veränderten CD8+-T-Zellen in der Zusammensetzung größer als 1:3 und kleiner als 3:1 ist.

2. Zusammensetzung zur Verwendung in einem Verfahren zur Behandlung oder zur Prävention von Krebs nach Anspruch 1; wobei das Verhältnis von CD4+-T-Zellen zu CD8+-T-Zellen in der Zusammensetzung größer als 1:3 und kleiner als 3:1 ist.

3. Zusammensetzung zur Verwendung in einem Verfahren zur Behandlung oder zur Prävention von Krebs nach Anspruch 1 oder Anspruch 2, wobei die Anzahl der gentechnisch veränderten CD4+-T-Zellen in der Zusammensetzung größer ist als eine Anzahl, die aus der aus 1 x 10⁶, 2,5 x 10⁶, 5 x 10⁶, 7,5 x 10⁶ und 9 x 10⁶ bestehenden Gruppe ausgewählt ist, und wobei die Anzahl der gentechnisch veränderten CD8+-T-Zellen in der Zusammensetzung größer als eine Anzahl ist, die aus der aus 1 x 10⁶, 2,5 x 10⁶, 5 x 10⁶, 7,5 x 10⁶ und 9 x 10⁶ bestehenden Gruppe ausgewählt ist.

4. Zusammensetzung zur Verwendung in einem Verfahren zur Behandlung oder zur Prävention von Krebs nach einem der Ansprüche 1 bis 3, wobei die Anzahl der gentechnisch veränderten CD4+-T-Zellen in der Zusammensetzung zwischen 10⁸ und 10¹² beträgt und wobei die Anzahl der gentechnisch veränderten CD8+-T-Zellen in der Zusammensetzung zwischen 10⁸ und 10¹² beträgt.

5. Zusammensetzung zur Verwendung in einem Verfahren zur Behandlung oder zur Prävention von Krebs nach einem der Ansprüche 1 bis 4, wobei jede der Anzahlen von gentechnisch veränderten CD4+-T-Zellen und die Anzahl der gentechnisch veränderten CD8+-T-Zellen zwischen 10¹⁰ und 10¹¹ beträgt.

6. Zusammensetzung zur Verwendung in einem Verfahren zur Behandlung oder zur Prävention von Krebs nach einem der Ansprüche 1 bis 5, wobei das Verhältnis von gentechnisch veränderten CD4+-T-Zellen zu gentechnisch veränderten CD8+-T-Zellen in der Zusammensetzung 1:1 beträgt.

7. Zusammensetzung zur Verwendung in einem Verfahren zur Behandlung oder zur Prävention von Krebs nach einem der Ansprüche 1 bis 6, wobei für das Molekül, das in der Lage ist, an das Antigen zu binden, eine Nucleotidsequenz kodiert, die eine Sequenz umfasst, die für eine Aminosäuresequenz kodiert, die von TCR-ζ abgeleitet ist, und/oder die eine Sequenz umfasst, die für eine Aminosäuresequenz kodiert, die von der CD28-costimulatorischen Signalisierungsdomäne abgeleitet ist.

8. Zusammensetzung zur Verwendung in einem Verfahren zur Behandlung oder zur Prävention von Krebs nach einem der Ansprüche 1 bis 7, wobei für das Molekül ein chimäres Genkonstrukt kodiert, das für ein variables Einzelkettenfragment eines Antikörpers (scFv), eine Transmembran- und eine zytoplasmatische Region einer CD28-Signalkette und eine zytoplasmatische Region eines T-Zellen-Rezeptors kodiert, und wobei das scFv in der Lage ist, das Antigen auf der Zielzelle zu binden.

9. Zusammensetzung zur Verwendung in einem Verfahren zur Behandlung oder zur Prävention von Krebs nach Anspruch 8, wobei der T-Zellen-Rezeptor TCR-ζ ist.

10. Zusammensetzung zur Verwendung in einem Verfahren zur Behandlung oder zur Prävention von Krebs nach Anspruch 8 oder Anspruch 9, wobei das chimäre Genkonstrukt weiters für eine membranproximale Gelenkregion von CD8 kodiert.

11. Zusammensetzung zur Verwendung in einem Verfahren zur Behandlung oder zur Prävention von Krebs nach einem der Ansprüche 1 bis 10, wobei das Antigen in oder auf einer Tumorzelle zu finden ist.

12. Zusammensetzung zur Verwendung in einem Verfahren zur Behandlung oder zur Prävention von Krebs nach Anspruch 11, wobei das Antigen aus erbB2, Cd19, Le^{y}, EGFR, PMSA, G250, NY-ESO1, CD20, Idiotyp Ig, p53, ras, CEA, MUC1, GD2 und HuD ausgewählt ist.

13. Zusammensetzung zur Verwendung in einem Verfahren zur Behandlung oder zur Prävention von Krebs nach einem der vorangegangenen Ansprüche, wobei die Behandlung oder die Prävention von Krebs ein Krebsrezidiv betrifft.

14. Zusammensetzung zur Verwendung in einem Verfahren zur Behandlung oder zur Prävention von Krebs nach einem der Ansprüche 1 bis 13, wobei Zellen in der Zusammensetzung aus einem Immungewebe oder einem Tumorgewebe des Individuums abgeleitet sind und das Verhältnis von CD4+-T-Zellen zu CD8+-T-Zellen in der Zusammensetzung im Vergleich zu dem Verhältnis von CD4+-T-Zellen zu CD8+-T-Zellen in dem Immungewebe oder dem Tumorgewebe des Individuums erhöht ist.

15. Zusammensetzung zur Verwendung in einem Verfahren zur Behandlung oder zur Prävention von Krebs nach einem der vorangegangenen Ansprüche, wobei das Immungewebe oder das Tumorgewebe aus der aus Blut, Milz, Thymus, Lymphknoten oder Knochenmark bestehenden Gruppe ausgewählt ist.

16. Zusammensetzung zur Verwendung in einem Verfahren zur Behandlung oder zur Prävention von Krebs nach Anspruch 15, wobei das Gewebe Blut oder ein Tumorgewebe ist.

17. Verwendung einer Zusammensetzung zur Herstellung eines Medikaments zur Behandlung oder zur Prävention einer Krebsart, wobei die Zusammensetzung Zellen umfasst, wobei die Zellen aus (a) einer Population von CD4+-T-Zellen bestehen, wobei die Population CD4+-T-Zellen umfasst, die gentechnisch verändert sind, um ein Molekül zu exprimieren, das in der Lage ist, an ein Antigen auf einer Zielzelle des Krebses zu binden; und (b) einer Population von CD8+-T-Zellen bestehen, wobei die Population CD8+-T-Zellen umfasst, die gentechnisch verändert sind, um das Molekül zu exprimieren, das in der Lage ist, an das Antigen auf der Zielzelle des Krebses zu binden; wobei das Verhältnis der gentechnisch veränderten CD4+-T-Zellen zu den gentechnisch veränderten CD8+-T-Zellen in der Zusammensetzung größer als 1:3 und kleiner als 3:1 ist und wobei die Behandlung oder die Prävention von Krebs die systemische Injektion der Zusammensetzung involviert.

18. Verwendung nach Anspruch 17, wobei die Zellen in der Zusammensetzung aus einem Immungewebe oder einem Tumorgewebe des Individuums abgeleitet sind und das Verhältnis von CD4+-T-Zellen zu CD8+-T-Zellen in der Zusammensetzung im Vergleich zu dem Verhältnis von CD4+-T-Zellen zu CD8+-T-Zellen in dem Immungewebe oder dem Tumorgewebe des Individuums erhöht ist.

19. Verwendung nach Anspruch 18, wobei das Immungewebe oder das Tumorgewebe aus der aus Blut, Milz, Thymus, Lymphknoten oder Knochenmark bestehenden Gruppe ausgewählt ist.

20. Verwendung nach einem der Ansprüche 17 bis 19, wobei das Gewebe Blut oder ein Tumorgewebe ist.

## Revendications

1. Composition destinée à être utilisée dans un procédé de traitement ou de prévention du cancer chez un sujet, le procédé comprenant une injection systémique de la composition, la composition comprenant des cellules, où les cellules consistent en (a) une population de cellules T CD4+, la population comprenant des cellules T CD4+ manipulées pour exprimer une molécule capable de se lier à un antigène sur une cellule cible du cancer ; et (b) une population de cellules T CD8+, la population comprenant des cellules T CD8+ manipulées pour exprimer la molécule capable de se lier à l'antigène sur la cellule cible du cancer ; dans laquelle le rapport des cellules T CD4+ manipulées sur les cellules T CD8+ manipulées dans la composition est supérieur à 1:3 et inférieur à 3:1.

2. Composition destinée à être utilisée dans un procédé de traitement ou de prévention du cancer selon la revendication 1, dans laquelle le rapport des cellules T CD4+ sur les cellules T CD8+ dans la composition est supérieur à 1:3 et inférieur à 3:1.

3. Composition destinée à être utilisée dans un procédé de traitement ou de prévention du cancer selon la revendication 1 ou la revendication 2, dans laquelle le nombre de cellules T CD4+ manipulées dans la composition est supérieur à un nombre sélectionné dans le groupe consistant en 1 x 10⁶, 2,5 x 10⁶, 5 x 10⁶, 7,5 x 10⁶, et 9 x 10⁶, et dans laquelle le nombre de cellules T CD8+ manipulées dans la composition est supérieur à un nombre sélectionné dans le groupe consistant en 1 x 10⁶, 2,5 x 10⁶, 5 x 10⁶, 7,5 x 10⁶, et 9 x 10⁶.

4. Composition destinée à être utilisée dans un procédé de traitement ou de prévention du cancer selon l'une quelconque des revendications 1 à 3, dans laquelle le nombre de cellules T CD4+ manipulées dans la composition est compris entre 10⁸ et 10¹² et dans laquelle le nombre de cellules T CD8+ manipulées dans la composition est compris entre 10⁸ et 10¹².

5. Composition destinée à être utilisée dans un procédé de traitement ou de prévention du cancer selon l'une quelconque des revendications 1 à 4, dans laquelle chacun du nombre de cellules T CD4+ manipulées et du nombre de cellules T CD8+ manipulées est compris entre 10¹⁰ et 10¹¹.

6. Composition destinée à être utilisée dans un procédé de traitement ou de prévention du cancer selon l'une quelconque des revendications 1 à 5, dans laquelle le rapport des cellules T CD4+ manipulées sur les cellules T CD8+ manipulées dans la composition est de 1:1.

7. Composition destinée à être utilisée dans un procédé de traitement ou de prévention du cancer selon l'une quelconque des revendications 1 à 6, dans laquelle la molécule capable de se lier à l'antigène est codée par une séquence de nucléotides comprenant une séquence codant pour une séquence d'acides aminés dérivée d'un TCR-ζ et/ou comprenant une séquence codant pour une séquence d'acides aminés dérivée du domaine de signalisation co-stimulateur de CD28.

8. Composition destinée à être utilisée dans un procédé de traitement ou de prévention du cancer selon l'une quelconque des revendications 1 à 7, dans laquelle la molécule est codée par une construction génique chimérique codant pour un fragment variable à chaîne unique d'un anticorps (scFv), une région transmembranaire et cytoplasmique d'une chaîne de signalisation de CD28 et une région cytoplasmique d'un récepteur des cellules T, et dans laquelle le scFv est capable de se lier à l'antigène sur la cellule cible.

9. Composition destinée à être utilisée dans un procédé de traitement ou de prévention du cancer selon la revendication 8, dans laquelle le récepteur des cellules T est le TCR-ζ.

10. Composition destinée à être utilisée dans un procédé de traitement ou de prévention du cancer selon la revendication 8 ou la revendication 9, dans laquelle la construction génique chimérique code en outre pour une région charnière proximale de membrane de CD8.

11. Composition destinée à être utilisée dans un procédé de traitement ou de prévention du cancer selon l'une quelconque des revendications 1 à 10, dans laquelle l'antigène est observé dans ou sur une cellule tumorale.

12. Composition destinée à être utilisée dans un procédé de traitement ou de prévention du cancer selon la revendication 11, dans laquelle l'antigène est sélectionné parmi erbB2, Cd19, Le^{y}, l'EGFR, le PMSA, G250, NY-ESO1, CD20, un idiotype d'Ig, p53, ras, l'ACE, MUC1, GD2 et HuD.

13. Composition destinée à être utilisée dans un procédé de traitement ou de prévention du cancer selon l'une quelconque des revendications précédentes, où le traitement ou la prévention du cancer est pour une récidive du cancer.

14. Composition destinée à être utilisée dans un procédé de traitement ou de prévention du cancer selon l'une quelconque des revendications 1 à 13, où les cellules dans la composition sont dérivées d'un tissu immunitaire ou d'un tissu tumoral du sujet et le rapport des cellules T CD4+ sur les cellules T CD8+ dans la composition est augmenté par comparaison au rapport des cellules T CD4+ sur les cellules T CD8+ dans le tissu immunitaire ou le tissu tumoral du sujet.

15. Composition destinée à être utilisée dans un procédé de traitement ou de prévention du cancer selon l'une quelconque des revendications précédentes, dans laquelle le tissu immunitaire ou le tissu tumoral est sélectionné dans le groupe consistant en du sang, la rate, le thymus, un ganglion lymphatique ou la moelle osseuse.

16. Composition destinée à être utilisée dans un procédé de traitement ou de prévention du cancer selon la revendication 15, dans laquelle le tissu est du sang ou un tissu tumoral.

17. Utilisation d'une composition pour la fabrication d'un médicament destiné au traitement ou à la prévention d'un cancer, la composition comprenant des cellules, où les cellules consistent en (a) une population de cellules T CD4+, la population comprenant des cellules T CD4+ manipulées pour exprimer une molécule capable de se lier à un antigène sur une cellule cible du cancer ; et (b) une population de cellules T CD8+, la population comprenant des cellules T CD8+ manipulées pour exprimer la molécule capable de se lier à l'antigène sur la cellule cible du cancer ; où le rapport des cellules T CD4+ manipulées sur les cellules T CD8+ manipulées dans la composition est supérieur à 1:3 et inférieur à 3:1, et dans laquelle le traitement ou la prévention du cancer implique une injection systémique de la composition.

18. Utilisation selon la revendication 17, dans laquelle les cellules dans la composition sont dérivées d'un tissu immunitaire ou d'un tissu tumoral du sujet et le rapport des cellules T CD4+ sur les cellules T CD8+ dans la composition est augmenté par comparaison au rapport des cellules T CD4+ sur les cellules T CD8+ dans le tissu immunitaire ou le tissu tumoral du sujet.

19. Utilisation selon la revendication 18, dans laquelle le tissu immunitaire ou le tissu tumoral est sélectionné dans le groupe consistant en du sang, la rate, le thymus, un ganglion lymphatique ou la moelle osseuse.

20. Utilisation selon l'une quelconque des revendications 17 à 19, dans laquelle le tissu est du sang ou un tissu tumoral.
